(19) Europäisches Patentamt
European Patent Office
Office européen des brevets

(11) Publication number: **0 263 594 B1**

(12) **EUROPEAN PATENT SPECIFICATION**

(45) Date of publication of patent specification: **24.06.92**

(51) Int. Cl.5: **A61K 31/445**

(21) Application number: **87307860.4**

(22) Date of filing: **04.09.87**

(54) **Anxiolytic compositions containing dioxopiperidine derivatives.**

(30) Priority: **08.09.86 GB 8621577**
**10.07.87 GB 8716339**

(43) Date of publication of application:
**13.04.88 Bulletin 88/15**

(45) Publication of the grant of the patent:
**24.06.92 Bulletin 92/26**

(84) Designated Contracting States:
**BE CH DE ES FR IT LI LU NL SE**

(56) References cited:
**EP-A- 0 216 555**
**EP-A- 0 216 555**
**EP-A- 0 229 444**
**FR-A- 2 212 147**
**US-A- 4 461 771**

**BIOCHEMICAL PHARMALOGY, vol. 35, no. 9, pages 1521-1526, Pergamon Press Ltd, GB; M.C. BOADLE-BIBER et al.: "AGN 2979 [3-(3-Methoxyphenyl)-3-(3-Dimethylaminopropyl)-4,4-Dimethylpiperidine-2,6-dione]. An inhibitor of the activation of tryptophan hydroxylase"**

**J. MED. CHEM., vol. 29, no. 8, August 1986, pages 1476-1482, American Chemical Society, Washington, D.C., US; J.S. NEW et al.: "Buspirone analogues. 2. Structure-activity relationships of aromatic imide derivatives"**

(73) Proprietor: **BRITISH TECHNOLOGY GROUP PLC**
**101 Newington Causeway**
**London SE1 6BU(GB)**

(72) Inventor: **Gittos, Maurice Ward**
**16 rue André Malraux**
**F-67115 Plobsheim(FR)**
Inventor: **Costall, Brenda**
**The Old Rectory**
**Addingham, North Yorkshire(GB)**

(74) Representative: **Davis, Norman Norbridge et al**
**BRITISH TECHNOLOGY GROUP 101 Newington Causeway**
**London SE1 6BU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to the use of certain 3-phenyl-3-aminoalkyl-4-methyl-2,6-dioxopiperidines as anxiolytic drugs. In particular, the invention provides the use of the said dioxopiperidines in the manufacture of anxiolytic medicaments and low dosage unit dose compositions comprising said dioxopiperidines.

The most widely prescribed anti-anxiety drugs are benzodiazepines angonists such as diazepam and it is known that these drugs act by interacting with a benzodiazepine receptor. When used in low doses they have virtually no side effects but their anti-anxiety effectiveness is often not sufficient. Increasing the dose to a normal effective one often produces side effects such as dizziness and sedation. These doses can also lead to memory impairment. Further, tolerance to their effect usually develops within four months of continuous use and there exists a substantial risk of addiction in many patients.

Benzodiazepine agonists are generally believed to exert their anxiolytic action by respectively enhancing the coupling function of the benzodiazepine receptor to the gamma-aminobutyric acid (GABA) receptor-chloride channel complex. It is also known that benzodiazepine agonists reduce the turnover of serotonin (5-hydroxytryptamine; 5HT) but the significance of this reduction has not previously been known.

It has surprisingly now been found that certain 3-phenyl-3-aminoalkyl-4-methyl-2,6-dioxopiperidines (as defined hereinafter) have strong anxiolytic activity.

GB 1455687 (also AU 480855, BE 808958, DE 23630526, FR 7346904, JP 6053014 and US 3963729) discloses that 3-phenyl-3-aminoalkyl-4- and/or 5-methyl-2,6-dioxopiperidine derivatives have central nervous system, especially antidepressant, activity. Said compounds include, inter alia, those of the following Formula A.

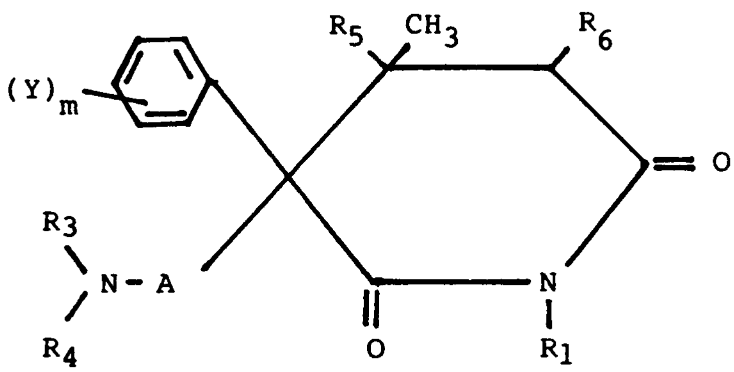

(A)

wherein:

$R_1$ represents hydrogen or $C_1$-$C_4$ alkyl;
$R_3$ represents hydrogen or $C_1$-$C_4$ alkyl;
$R_4$ represents $C_1$-$C_4$ alkyl;
$R_5$ and $R_6$ independently represent hydrogen or methyl;
A represents $C_1$-$C_6$ alkylene;
m is 0 to 3; and
Y is hydroxy, $C_1$-$C_4$ alkoxy, $C_1$-$C_4$ alkyl, $C_1$-$C_4$ hydroxyalkyl, halogen or trifluoromethyl.

The dose level specified for administration of the compounds is 0.1 to 100 mg/kg using pharmaceutical compositions containing 1 to 1000 mg per unit dose.

It also has been disclosed in U.S. 4,461,771 that compounds of Formula A, in which $R_1$ represents hydrogen; $R_3$ and $R_4$ independently represent methyl or ethyl; $R_5$ represents methyl; $R_6$ represents hydrogen; A represents ethylene or propylene; m is 1 or 2; and Y is in a meta position and independently represents hydroxy or $C_1$-$C_2$ alkoxy, are believed to reduce in vitro the activity of tryptophan hydroxylase by blocking the depolarization-induced activation of the enzyme in the brain stem and hence are of potential use in the prophylactic treatment of the stressful disorder migraine. The dose level specified for this treatment is 0.01 to 10 mg/kg, especially 0.1 to 3 mg/kg, using pharmaceutical compositions containing 0.1 to 200 mg, usually 1 to 100 mg, per unit dose. More recently, it has been reported that at least one compound of Formula A (viz 3-(3′-methoxy-phenyl)-3-(3″-N,N-dimethylaminopropyl)-4,4-dimethyl-2,6-dioxo piperidine; AGN 2979) also blocks in vitro the activation of tryptophan hydroxylase resulting from exposure of brain stem slices to metabolic inhibitors or methylxanthines or induced by incubation of supernatant preparations of the enzyme under phosphorylating conditions (Boadle-Biber, M.C. et al Biochem, Pharmacol. 35, 1521-6, (1986)). However, it also has been reported that AGN 2979 has no significant effect in vitro upon the unactivated enzyme (Boadle-Biber, M.C. et al supra).

Further, it has recently been disclosed in GB 2181346A that compounds of Formula A, in which $R_1$ represents hydrogen; $R_3$ and $R_4$ independently represent methyl or ethyl; A represents ethylene or propylene; m is 1 or 2; and each Y is in a meta position and independently represents hydroxy or $C_1$-$C_2$ alkoxy, are believed to reduce the turnover of 5-hydroxytryptamine (5HT) resulting from inhibiting the activity of tryptophan hydroxylase. They are reported to have anxiolytic activity, antagonize the anxiogenic activity of benzodiazepines inverse agonists, reduce chronic abnormally high brain levels of 5HT or its metabolite 5-hydroxy-indoleacetic acid, and have antibacterial and antiviral activity. GB-A-2181346 (corresponding to EP-A-0216555) was published in pursuance of U.K. Patent Application No. 8621577 filed 8th September 1986 and claiming priority from U.K. Patent Applications Nos. 8522455 (filed 11th September 1985), 8603909 (filed 17th February 1986) and 8603910 (also filed 17th February 1986). Originally, it was thought that the disclosed compounds were not themselves anxiolytic because they have virtually no action at benzodiazepine receptors and that they acted via some unknown pharmacological mechanism to potentiate the anxiolytic activity of benzodiazepine receptors. Their anxiolytic activity was disclosed for the first time in U.K. Patent Application No. 8621577. At that time, the compounds were believed to be active in the range 0.1 to 20 mg/kg, especially 0.5 to 10 mg/kg and hence pharmaceutical compositions containing 10 to 500 mg, especially 10 to 100 mg, were proposed. However, it has now surprisingly been found that the compounds are active at much lower dose levels, down to nanogram/kg amounts.

According to a first aspect of the present invention, there is provided the use in an amount of $10^{-6}$ to $10^{-1}$ mg per unit dose in the manufacture of a medicament for the treatment of anxiety of a compound of the following Formula I.

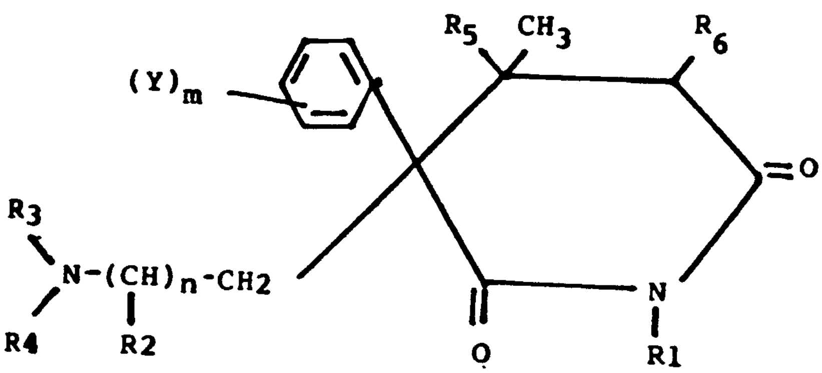

(I)

wherein:

$R_1$ represents hydrogen or $C_1$-$C_4$ alkyl;

n is 1 or 2;

$R_2$ represents hydrogen or methyl, provided that at least one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl;

m is 0 to 3; and

each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

or a pharmacologically acceptable salt thereof.

According to a second aspect of the invention, there is provided a pharmaceutical composition in unit dose form comprising, with a pharmaceutically acceptable diluent or carrier an amount of $10^{-6}$ to less than $10^{-1}$ mg per unit dose of a compound of the following Formula I:

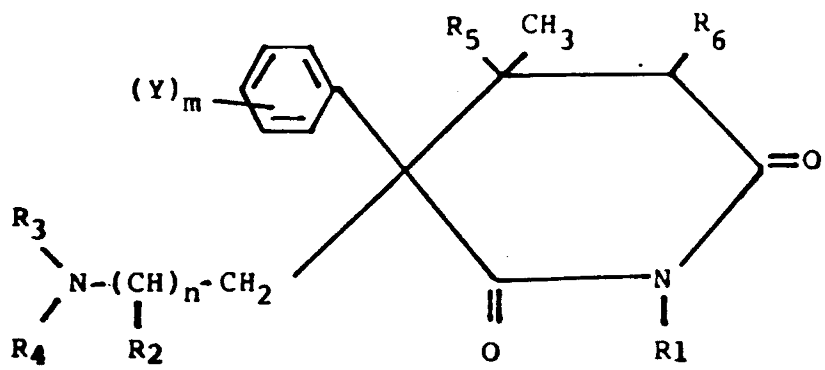

(I)

wherein:

$R_1$ represents hydrogen or $C_1$-$C_4$ alkyl;

n is 1 or 2;

$R_2$ represents hydrogen or methyl, provided that at least one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl;

m is 0 to 3; and

each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

or a pharmacologically acceptable salt thereof.

The compounds of Formula I can be prepared in the manner disclosed in GB 1455687. They exist as optical isomers and can be used in racemate form or as individual (+) or (-) isomers. Presently, the (-) isomer is preferred.

As mentioned above the compounds of Formula 1 have anxiolytic activity and hence are useful in the treatment of anxiety. At least some of the compounds are ten orders ($10^5$) more potent than diazepam and exhibit an effective dose range in the order of millions fold (eg. $10^{-4}$-$10^2$ mg/kg). They cause dose related anxiolytic effects and do not cause sedation at high doses ($10^2$ mg/kg).

The compounds of Formula I can be administered in various manners to achieve the desired anxiolytic effect. The compounds can be administered enterally or parenterally to the patient being treated. Oral administration is likely to be the preferred route in most circumstances but injection, especially subcutaneously or intravenously, will be preferred in some circumstances.

The amount of compound administered will vary and can be any anti-anxiety effective amount. Depending upon the patient and the mode of administration, the amount of compound administered may vary over a wide range to provide from $10^{-7}$ to $10^2$ mg/kg, usually $10^{-5}$ to $10^2$ mg/kg, especially $10^{-4}$ to $10^2$ mg/kg, of body weight of the patient per unit dose. Unit doses of these compounds can contain, for example, from $10^{-6}$ mg to 500 mg, usually $10^{-4}$ to $10^2$ mg, especially $10^{-3}$ to $10^2$ mg of the compound and may be administered, for example, from 1 to 4 times daily.

The term "unit dosage form" is used herein to mean a single or multiple dose form containing a quantity of the active ingredient in admixture with or otherwise in association with a diluent or carrier, said quantity being such that one or more predetermined units are normally required for a single therapeutic administration. In the case of multiple dose forms such as liquids or scored tablets, said predetermined unit will be one fraction, such as a 5 ml (teaspoon) quantity of a liquid or a half or quarter of a scored tablet, of the multiple dose form.

The compounds of Formula I have virtually no action at benzodiazepine receptors. The capacity of a selected number of compounds of Formula I to displace triturated flunitrazepam from benzodiazepine receptors has been measured with the results set forth in Table I below:-

TABLE 1

| | COMPOUND OF FORMULA I | | | | |
|---|---|---|---|---|---|
| | 2979 | 3222 | 2939- | 3181 | DIAZEPAM* |
| IC50(μM) [3H]Flunitrazepam Binding | 350 | 1300 | 9000 | 6700 | 0.014 |

* 7-chloro-1,3-dihydro-1-methyl-5-phenyl-2H-1,4-benzodiazepine-2-one

The compounds of general Formula I can have the phenyl moiety substituted in one or both meta positions by $C_1$-$C_2$ alkyl, $C_1$-$C_2$ hydroxyalkyl, halogen, trifluoroethyl, or, preferably, hydroxy or $C_1$-$C_2$ alkoxy. Additionally or alternatively, the phenyl moiety can be substituted in the para position by the aforementioned groups other than hydroxy and alkoxy. It is presently preferred that the substituent(s) should be hydroxy or, especially, methoxy. It is also preferred that one or both meta positions are substituted and that, when there are two substituents, they should be the same.

The amino group of the compounds of Formula I can be secondary or tertiary having methyl or ethyl groups attached to the nitrogen atom. Dimethylamino presently is preferred. The amino group is connected to the piperidine ring by a divalent ethylene (i.e. n = 1) or trimethylene (i.e. n = 2) radical optionally substituted on a carbon atom not adjacent said ring with a methyl group. Presently, unsubstituted trimethylene is preferred.

The piperidine ring of the compounds of Formula I is substituted in the 4-position with methyl and optionally by one or two further methyl groups in the 4 and/or 5 positions. It is presently preferred that there is one further methyl group in the 4 or 5 position, especially in the 4-position.

The ring nitrogen atom of the piperidine ring can be substituted with a $C_1$-$C_4$ alkyl group but it is presently preferred that said nitrogen atom is unsubstituted.

The $C_1$-$C_2$ alkyl groups or moieties referred to herein are methyl or ethyl; methyl presently being preferred. The $C_3$-$C_4$ alkyl groups which may be substituents on the nitrogen atom of the piperidine ring can be straight or branched chain but the straight chain n-propyl or n-butyl groups presently are preferred. The halogen substituent(s) in the phenyl ring can be chlorine, bromine or fluorine; chlorine presently being preferred.

The presently preferred compounds of Formula I are those of the following Formula IA.

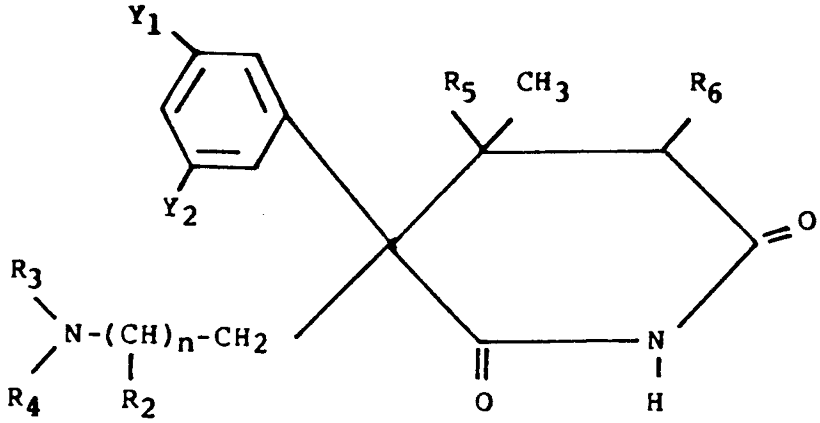

(IA)

wherein:

n is 1 or 2;

$R_2$ represents hydrogen or methyl, provided that at least one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl; and

$Y_1$ and $Y_2$ independently represent hydrogen, hydroxy or $C_1$-$C_2$ alkoxy,

or a pharmacologically acceptable salt thereof.

The presently especially preferred compounds of Formula 1A are those of the following Formula IB.

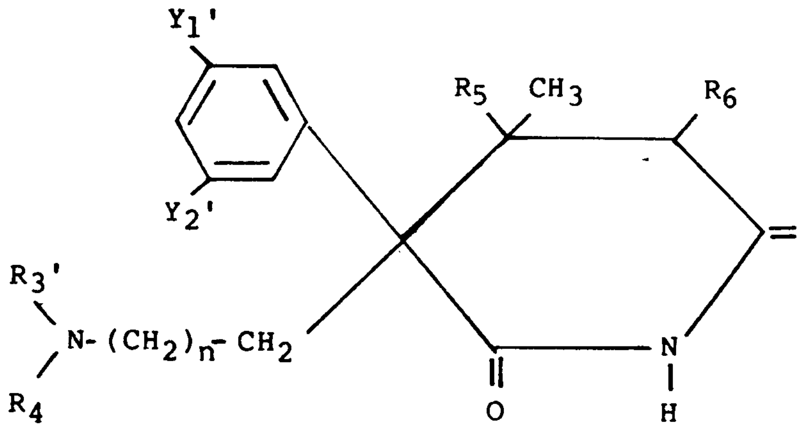

(IB)

wherein:

n is 1 or 2;

$R_3'$ and $R_4$ independently represent $C_1$-$C_2$ alkyl;

$R_5$ and $R_6$ independently represent hydrogen or methyl;

$Y_1'$ represents hydroxy or $C_1$-$C_2$ alkoxy; and

$Y_2'$ represents hydrogen, hydroxy or $C_1$-$C_2$ alkoxy,

or a pharmacologically acceptable salt thereof.

Examples of compounds of Formula IB include the following:

3-(3'-methoxyphenyl)-3-(2"-N,N-dimethylaminoethyl)-4,4-dimethyl-2,6-dioxopiperidine
3-(3'-methoxyphenyl)-3-(3"-N,N-dimethylaminopropyl)-4,4-dimethyl-2,6-dioxopiperidine (AGN 2979);
3-(3'-methoxyphenyl)-3-(2"-N,N-diethylaminoethyl)-4,4-dimethyl-2,6-dioxopiperidine;
3-(3'-methoxyphenyl)-3-(3"-N,N-diethylaminopropyl)-4,4-dimethyl-2,6-dioxopiperidine;
3-(3'-hydroxyphenyl)-3-(2"-N,N-dimethylaminoethyl)-4,4-dimethyl-2,6-dioxopiperidine;
3-(3'-hydroxyphenyl)-3-(3"-N,N-dimethylaminopropyl)-4,4-dimethyl-2,6-dioxopiperidine;
3-(3'-methoxyphenyl)-3-(2"-N,N-dimethylaminoethyl)-4,5-dimethyl-2,6-dioxopiperidine (AGN 2939);
3-(3'-methoxyphenyl)-3-(3"-N,N-dimethylaminopropyl)-4,5-dimethyl-2,6-dioxopiperidine (AGN 3181);
3-(3'-ethoxyphenyl)-3-(3"-N,N-dimethylaminopropyl)-4,4-dimethyl-2,6-dioxopiperidine;
3-(3'-ethoxyphenyl)-3-(3"-N,N-diethylaminopropyl)-4,4-dimethyl-2,6-dioxopiperidine;
3-(3',5'-dimethoxyphenyl)-3-(3"-N,N-dimethylamino-propyl)-4,4-dimethyl-2,6-dioxopiperidine (AGN 3222);
3-(3',5'-dimethoxyphenyl)-3-(2"-N,N-dimethylamino-ethyl)-4,4-dimethyl-2,6-dioxopiperidine;
3-(3′,5′-dimethoxy phenyl)-3-(3″-N,N-dimethylaminopropyl)-4,5-dimethyl-2,6-dioxopiperidine; and
3-(3′,5′-dimethoxyphenyl)-3-(2″-N,N-dimethylamioethyl)-4,5-dimethyl-2,6-dioxopiperidine;

Examples of other compounds of Formula I include:-

3-phenyl-3-(2′-N,N-dimethylaminoethyl)-4-methyl-2,6-dioxopiperidine;
3-phenyl-3-(2′-N,N-dimethylaminoethyl)-4,4-dimethyl-2,6-dioxopiperidine;
3-phenyl-3-(2′-N,N-dimethylaminoethyl)-4,5-dimethyl-2,6-dioxopiperidine;
3-phenyl-3(3′-N,N-dimethylaminoethyl)-4,4-dimethyl-2,6-dioxopiperidine;
3-(4′-chlorophenyl)-3(3″-N,N-dimethylaminopropyl)-4,4-dimethyl-2,6-dioxopiperidine; and
3-phenyl-3(2′ N-methylaminoethyl)-4,4-dimethyl-2,6-dioxopiperidine.

The compounds of Formula I may be administered in free base form, as an alkali metal or alkaline earth metal salt or as a pharmaceutically acceptable acid addition salt with the proviso that an alkali metal or alkaline earth metal salt is not normally combined with an acid solution salt except in a layer formulation. Representative acid addition salt forms include organic acid salt forms such as the maleate and methane

sulphonate and mineral acid salt forms such as the hydrochloride and perchlorate.

The pharmaceutical formulations in which form the active compounds of the invention will normally be utilized are prepared in a manner well known per se in the pharmaceutical art and usually comprise at least one active compound of Formula I in admixture or otherwise in association with a pharmaceutically acceptable carrier or diluent therefor. For making those formulations the active ingredient will usually be mixed with a carrier, or diluted by a diluent, or enclosed or encapsulated in a capsule, sachet, cachet, paper or other container. A carrier or diluent may be solid, semi-solid or liquid material which serves as a vehicle, excipient or medium for the active ingredient. Suitable carriers or diluents are well known per se.

The formulations may be adapted for enteral or parenteral use and may be administered to the patient in the form of tablets, capsules, dragees, suppositories, syrups or suspensions.

The formulations may be in delayed or sustained release form.

Aside from the active agents the compositions may contain pharmaceutically inert organic or inorganic adjuvants, optionally granulating agents, binding agents lubricants, dispersing agents, wetting agents and preservatives. Moreover, the pharmaceutical compositions may contain colouring, flavouring and sweetening substances. Adjuvants for the production of tablets may be e.g. calcium carbonate, lactose micro-crystalline cellulose, mannitol or talc. Starch and alginic acid or micro-crystalline cellulose may be used as granulating and disintegrating agents, starch, polyvinylpyrrolidone and gelatine may be used as binding agents and magnesium stearate, stearic acid, colloidal silica and talc as lubricants. Tablet formulation may be coated or uncoated, the coating having the purpose of delaying the disintegration and absorption in the gastrointestinal tract. Suitable suspending agents for the production of liquid administration forms are e.g. methyl cellulose and sodium alginate. Capsule formulation may contain the active agents on their own or together with an inert solid diluent e.g. calcium phosphate, corn starch, lactose, or mannitol.

The invention is illustrated in the following Examples, some of which Examples relate to unit dose levels above those of the present invention.

## EXAMPLE 1

### Tablet Formulation

Tablets each having the following composition are prepared by conventional techniques:

| | | mg/tablet |
|---|---|---|
| (a) | Compound AGN 2979 base | 1 |
| (b) | Lactose | 51.5 |
| (c) | Maize starch dried | 45 |
| (d) | Magnesium stearate | 1.5 |

## EXAMPLE 2

### Suppository Formulation

| | | mg/suppository |
|---|---|---|
| (a) | Compound AGN 2979 HCl | 10 |
| (b) | Oil of Theobroma (cocoa butter) | 990 |

The compound (a) is powdered and passed through a BS No. 100 sieve and triturated with molten oil of Theobroma at 45° C to form a smooth suspension. The mixture is well stirred and poured into moulds each of nominal 1 G capacity to produce suppositories.

EXAMPLE 3

Tablet Formulation

| | | |
|---|---|---|
| (a) | Compound AGN 2979 base | 10mg |
| (b) | Wheat starch | 7g |
| (c) | Lactose | 20g |
| (d) | Magnesium Stearate | 1g |

The mixture is compressed into 1000 tablets each weighing 138 mg.

EXAMPLE 4

Pill Formulation

| | | per pill |
|---|---|---|
| (a) | Compound AGN 2979 HCl | 10mg |
| (b) | Corn starch | 45mg |
| (c) | Liquid glucose | 7ml |

The pills are prepared by blending the active ingredient (a) and the corn starch, then adding the liquid glucose with thorough kneading to form a plastic mass for which the pills are cut and formed.

EXAMPLE 5

Gelatine Capsule Formulation

| | | per capsule |
|---|---|---|
| (a) | Compound AGN 2979 HCl | 2.5mg |
| (b) | Talc | 70mg |

A capsule is prepared by passing dry powdered active ingredient (a) and powdered talc in the above proportions through a fine mesh screen and mixing the well. The powder is then filled into hard gelatin capsules at a net fill of 72.5mg per capsule.

EXAMPLE 6

Experimental animals

Naive male albino BKW mice, 25-30g, were used in all experiments. 10 mice were normally housed in each cage and given free access to food and water. The mice were kept on a 12h light and 12h dark cycle with lights off at 8.00 am and on at 8.00 pm.

Anti-anxiety test

The apparatus used for the detection of changes in anxiety consisted of an open topped box (81 x 36 x 27cm high) one third painted black and illuminated under a dim red light (1 x 60W) and partitioned from the remainder of the box which was painted white and brightly illuminated with a 100W light source located 17cm above the box. Access between these areas was enabled by means of a 7.5 x 7.5. cm opening located at floor level in the centre of the partition. The floor area was marked by lines into 9cm squares. The test was conducted between 13.00h and 18.00h in a quite, darkened room illuminated with red light only. Animals were thus taken in a dark container from a dark holding room to the dark testing room.

Animals that had received drug or vehicle injections were placed individually into the centre of the white area and their behaviour observed over a 5 minute period by remote video recording. Four behavioural parameters were noted every minute; viz the number of exploratory rearings in the white and black sections, the number of line crossings in the white and black areas, the number of transitions between the white and black or black and white areas, and the time spent in the white and black areas. Experimenters remained blind to drug treatment throughout, with the code only being broken after analysis was complete.

Experimental design

Animals were used in treatment groups of 5 and vehicle controls were run on each day of testing. Results were analysed using Single- Factor Analysis of Variance and, where appropriate, followed by Dunnett's procedure for comparing all treatments with control.

Drugs

(±) AGN 2979 was prepared in distilled water and diazepam (Roche) in the minimum quantity of polyethylene glycol (less than 25%) was prepared to volume with distilled water. Doses (expressed as the base) were administered as a 60 min pretreatment in a volume of 1ml/100g body weight by intraperitoneal (diazepam) or subcutaneous (AGN 2979) injection.

The results are indicated in accompanying Figures 1, 2 and 3 and discussed below.

Figure 1 shows the changes in rearing behaviour and line crossings (locomotion) in the white and black sections of the box for diazepam given intraperitoneally. C indicates the responses of vehicle-treated, control animals. n = 5, except for C where n = 15. S.E.M.s less than 11.6%. Significant increases in responding are indicated as *P less than 0.001, significant decreases as +P less than 0.05-P less than 0.001 (one-way ANOVA followed by Dunnett's 't' test).

Figure 2 shows the changes in rearing behaviour and line crossings (locomotion) in the white and black sections of the box for AGN 2979 given subcutaneously into the back neck region. C indicates the responses of vehicle-treated, control animals. n = 5, except for C where n = 15. S.E.M.s less than 7.3%. Significant increases in responding are indicated as +P less than 0.001, significant decreases as *P less than 0.01-P less than 0.001 (one-way ANOVA followed by Dunnett's 't' test).

Figure 3 shows the changes in the time spent by mice in exploration of the black section of the box for AGN 2979 injected subentaneously into the back neck region. C indicates the response of vehicle-treated control animals. n = 5, except for C where n = 15. S.E.M.s on original data less than 6.6%. Significant decreases in the time spent in the black sections are indicated as *P less than 0.01 - less than 0.001 (one-way ANOVA followed by Dunnett's 't' test). General observations

Within the test situation vehicle-treated animals displayed a characteristic behavioural profile which is typified as follows:

(1) an approximately equal time spent in each section of the test area;

(2) a transition rate between the two areas in the order of 8.3 ± 0.1/5 min;

(3) a significant difference between locomotion in the white section (33.7 ± 3.0/5 min) and line crossing in the black (53.5 ± 4.9/5 min)

(4) a marked increase in rearing in the black section (49.7 ± 3.2/5 min) as compared to the incidence of this behaviour in the white (22.1 ± 1.5/5 min).

These data indicate that, under test conditions, animals display a marked preference for activity in the black section of the test area, possibly induced by the aversive properties of the brightly-lit white painted area. Intuitively these findings may have been expected in view of the nocturnal nature of the species employed. Preference for the black section is most clearly demonstrated by measures of locomotion and rearing.

Detailed initial studies showed that the responses of vehicle treated animals were not significantly different (P greater than 0.05) from those derived above for non-treated control animals. Hence, only the responses obtained from vehicle-treated animals are given as control data.

Modification of exploratory behaviour by diazepam

Diazepam (0.125-5.0mg/kg) caused a 2 to 4 fold increase in rearing and a 1.5-2 fold increase in line crossings in the white area. A lower dose of 0.063mg/kg was ineffective, emphasising the all or none nature of the response. A higher dose of 10mg/kg caused sedation and a reduction of rearings and line crossings. The increased rearings and line crossings in the white area was mirrored by a reduction in these behaviours in the black area (Fig. 1).

Modification of exploratory behaviour by AGN 2979

AGN 2979 was as effective as diazepam to enhance rearings and line crossings in the white area and, as seen with diazepam, such changes correlated with reduced rearings and line crossings in the dark area (Fig. 2). The most notable features of the action of AGN 2979 were (a) its exceptional potency (0.00001 - 100.0 mg/kg) to increase behavioural measures in the white section, (b) the exceptional dose range at which the responses could be obtained (doses were administered over a range to the order of 10 million ($10^6$), and (c) the dose related effects of AGN 2979 which contrasts with the all-or-none response of diazepam. Additionally mice treated with AGN 2979 showed a marked reduction in time spent in the black section (Fig. 3) and, importantly, the effects of AGN 2979 were achieved in the absence of sedation.

EXAMPLE 7

The procedure of Example 6 was repeated except that separate groups of mice were tested 0.5, 1, 2, 4 and 6 h after subcutaneous administration of 0.1 mg/kg of 10 mg/kg (±) AGN 2979 in distilled water. The results were analysed using Single-Factor Analysis of Variance by Dunnetts procedure for comparing all treatments with vehicle-treated mice as control. They are indicated in accompanying Figures 4 and 5 and discussed below.

Figure 4 shows the changes in rearing behaviour and line crossings (locomotion) in the black and white sections of the box for 0.1 mg/kg AGN 2979 given subentaneously into the back neck region. C indicates that responses of vehicle-treated control animals. n = 5. S.E.M.s given. Significant increases in responding are indicated as *P less than 0.05, **P less than 0.01-P less than 0.001, significant decreases as + + P less than 0.01-P less than 0.001 (one-way ANOVA followed by Dunnett's "t" test).

Figure 5 corresponds to Figure 4 for the subcutaneous injection of 10 mg/kg AGN 2979 into the back neck region.

The anxiolytic action of AGN 2979, characterised by increased rearing and locomotion in the white section of the test box to which mice are normally averse, with corresponding decrease in behaviour in the black compartment, was seen to develop within 30 minutes of administering 0.1 mg/kg AGN 2979, although the maximum effect was not attained for 1h. The anxiolytic action was then maintained for the following administration, with return to control values after 6h (Fig.4). A similar course of onset and duration was recorded for 10 mg/kg AGN 2979 but, using this dose, the anxiolytic action was more established after 30 minutes than at the lower dose, and the maximum effect was well sustained over a 4h period. However, as observed at the lower dose, the anxiolytic activity was no longer detectable after 6h.

EXAMPLE 8

Male Sprague-Dawley rats, 225-275g, were normally housed in groups of 5 and kept on a 12 h light/dark cycle with lights on at 08.00 h. Tests were conducted between 13.00 and 18.00 h in a brightly illuminated room.

The apparatus used for the detection of changes in social interaction consisted of an opaque white Perspex (TM) open-topped box (45 x 32 cm x 20 cm high) with 15 x 16 cm areas marked on the floor. Two native rats, taken from separate housing cages, were placed in the test box (with a 100W bright white illumination 17 cm above) and their behaviour observed over a 10 minute period by remote video recording. Social interaction between the animals was determined by timing (seconds) sniffing of partner, crawling under or climbing over partner, grooming partner, genital investigation of partner, following partner. In addition, exploratory locomotion was measured as the number of crossings of the lines marked on the test box floor.

The experimental design was to use rats in groups of 6 (i.e. 3 pairs). (±) AGN 2979 was prepared in distilled water and diazepam (Roche) in the minimum quantity of polyethylene glycol ( 25%) prepared to volume with distilled water. Doses are expressed as the base and were administered by the intraperitoneal

route as a 40 in pretreatment in a volume of 1 ml/kg body weight.

The results are indicated in accompanying Figures 6 and 7 and discussed below.

Figure 6 shows the effects of diazepam on rat social interaction (seconds in 10 minutes) and exploratory locomotion (number of line crossings/10 minutes) of paired rats placed in an observation box. n = 6. S.E.M.s are given. Significant increase in social interaction is indicated as *P less than 0.001 (one-way ANOVA followed by Dunnett's t test). indicates sedation.

Figure 7 shows the effects of AGN 2979 on rat social interaction (seconds in 10 minutes) and exploratory locomotion (number of line crossings/10 min) of paired rats placed in an observation box. n = 6. S.E.M.s are given. Significant increase in social interaction is indicated as *P less than 0.01 - P less than 0.001 (one-way ANOVA followed by Dunnett's t test).

EXAMPLE 9

The procedure of Example 6 was repeated except for the following differences:-

a) Dose-ranging experiment

Mice were given the following oral doses of (±) AGN 2979;
0.0001, 0.001, 0.1, 1.0, 10.0, 100.0mg/kg p.o. Tests were carried out 90 minutes after dosing.

b) Duration of action experiment

Thirty-five mice were given 100mg/kg (±) AGN 2979 p.o. and groups of 5 mice were studied at the following times after dosing:
1, 2, 4, 6, 10, 16, 22 hours

Three vehicle control groups were studied at intervals throughout the time of the experiment.

AGN 2979 was prepared in distilled water. Doses are expressed as the base and were administered by gavage in a volume of 2ml/100g body weight.

The results are indicated in accompanying Figures 8 and 9 and discussed below.

Figure 8 shows the changes in rearing behaviour and line crossings (locomotion) in the white and black sections of the box. C indicates the responses of vehicle-treated control animals. n = 5 for each group. Significant increases in responding are indicated as + p less than 0.01-p less than 0.001 (one-way ANOVA followed by Dunnett's 't' test).

Figure 9 shows the changes in rearing behaviour and line crossings (locomotion) in the white and black sections of the box. C indicates the responses of vehicle-treated control animals. n = 5 for each group. Significant increases in responding are indicated as + p less than 0.001; significant decreases as x p less than 0.01-p less than 0.001 (one-way ANOVA followed by Dunnett's 't'-test).

General Observations

Within the present test situation vehicle-treated animals displayed a characteristic behavioural profile which is typified as follows:

(1) an approximately equal time spent in each section of the test area;

(2) a transition rate between the two area in the order of 8.3 ± =.1/5 min;

(3) a significant difference between locomotion in the white section 38.4 ± 3.8/5 min) and line corssing in the black 51.2 ± 5.3/5 min);

(4) a marked increase in rearing in the black section 50.7 ± 5.2/5 min) as compared to the incidence of this behaviour in the white 24.6 ± 2.7/5 min).

The anxiolytic action of AGN 2979, characterised by increased rearing and locomotion in the white section of the test box to which mice are normally averse, with corresponding decreases in behaviour in the black compartment, was seen at the lowest dose used (0.0001 mg/kg p.o.). The effect was found to be dose-related with a near-maximal effect seen at 1.0-10.0 mg/kg p.o. After a dose of 100mg/kg p.o., the anxiolytic action was shown to develop within 1 hour of administration. The effect was maximal 1 hour post-dosing and was maintained at this level for at least 10 hours. The anxiolytic effect, while still significant, was beginning to decline at 16 hours and there was a return to control values 22 hours after dosing.

EXAMPLE 10

The procedure of Example 6 was repeated to determine the effects of abrupt withdrawal from long term treatment with diazepam (2.5 and 10 mg/kg) and (±) AGN 2979 (0.1 and 10 mg/kg). The experimental protocols were as follows:

1. Diazepam 10 mg/kg was given i.p. acutely or twice daily for 7 days. This dose of diazepam is the lowest to cause sedative problems on acute treatment, but tolerance to this rapidly develops on long-term treatment and, therefore, this dose was selected as the highest which could be given long-term for maintenance of a good anxiolytic action without complicating problems of sedation. The effects of acute treatment were included in the studies only for the purpose of comparison of anxiolytic response to short and long-term treatment.

Animals were tested for withdrawal responding 8h after the last treatment with diazepam (i.e. 8h after the 2nd dose on the 7th day). Assessments of the action of diazepam acutely were made 45 min after treatment, and to determine the effects of long-term treatment with diazepam, animals were tested 45 min after the administration of the 2nd dose of diazepam on the 7th day.

2. The above protocol was repeated using 2.5 mg/kg diazepam and 0.1 and 10 mg/kg AGN 2979. In order to ensure that the withdrawal from AGN 2979 was not associated with delayed problems, separate groups of mice were used to assess potential withdrawal effects 8 hr, 24 hr, 48 hr, 96 hr, 144 hr and 10 days after administration of the last dose of AGN 2979.

The results are indicated in the accompanying Figures 10 to 13 and are discussed below.

Figure 10 shows the anxiolytic action of 2.5 mg/kg diazepam given acutely or chronically (2.5 mg/kg i.p. given b.d., tested on day 7) and the effects of withdrawal measured 8, 48 and 96 hr after administration of the last dose of diazepam. C indicates the responses of vehicle-treated control animals receiving a single injection of vehicle or those receiving vehicle twice daily for 7 days, P greater than 0.005). n = 6. S.E.M.s less than 12.4%. *, + P less than 0.001 (Dunnett's t test).

Figure 11 shows the anxiolytic action of 10.0 mg/kg diazepam given acutely or chronically (10.0 mg/kg i.p. given b.d., tested on day 7) and the effects of withdrawal measured 8, 48 and 96 hr after administration of the last dose of diazepam. C indicates the responses of vehicle-treated control animals receiving a single injection of vehicle or those receiving vehicle twice daily for 7 days, P greater than 0.05).
n = 6. S.E.M.s less than 12.2%. *, + P less than 0.01-P less than 0.001 (Dunnett's t test). ° indicates sedation.

Figure 12 shows the anxiolytic action of 0.1 mg/kg AGN 2979 given acutely or chronically (0.1 mg/kg i.p. given b.d., tested on day 7) and the effects of withdrawal measured 8, 48 and 96 hr after administration of the last dose of AGN 2979. C indicates the responses of vehicle-treated control animals receiving a single injection of vehicle or those receiving vehicle twice daily for 7 days, P greater than 0.05).
n = 6. S.E.M.s less than 11.7%. *, + P less than 0.001 (Dunnett's t test).

Figure 13 shows the anxiolytic action of 10 mg/kg AGN 2979 given acutely or chronically (10.0 mg/kg i.p. given b.d., tested on day 7) and the effects of withdrawal measured 8, 48 and 96h after administration of the last dose of AGN 2979. C indicates the responses of vehicle-treated control animals receiving a single injection of vehicle or those receiving vehicle twice daily for 7 days, P greater than 0.05). n = 6. S.E.M.s less than 12.1%. *, + P less than 0.001 (Dunnett's t test).

Acute treatment with diazepam, 2.5 mg/kg i.p., caused an anxiolytic response in the mouse test procedure. This was characterised by reduced aversion to the white, brightly-lit area of the test box (rearings and line crossings in the white significantly increased). The increased behavioural responding in the white area was mirrored by reduced behaviour in the black (Fig. 10). This anxiolytic action of diazepam was associated with a prolonged latency of entering the black section of the box, and with a reduced % of time spent in the dark section. In this, as in other experiments reported here, transitions between the two compartments of the test box were not significantly modified by drug treatment.

The anxiolytic action of 2.5 mg/kg i.p. diazepam was maintained on subchronic treatment (Fig. 10). However, when treatment was withdrawn a clear anxiogenesis developed which was characterised by reduced latency to enter the black section of the test box, increased rearings and line crossings in the black, and increased time spent in the black. The anxiogenesis was apparent within 8 h of the last treatment with diazepam and persisted for 48 h (Fig. 10).

Similar data was obtained using a higher dose of diazepam, 10 mg/kg i.p. The major differences seen between the high and low dose diazepam treatment was (1) the sedative action of the former which was apparent on acute treatment (tolerance to this sedative action developed within 3 days of continued b.d. treatment), and (2) the more clear persistence of the withdrawal anxiogenesis for 48 hr after ceasing treatment (Fig. 11).

The anxiolytic action of AGN 2979 was seen on acute treatment with 0.1 mg/kg (Fig. 12) and 10 mg/kg (Fig. 13): sedation was not observed. The nature of the anxiolytic action of AGN 2979 was, like diazepam,

characterised by a reduced latency to enter the black section of the test box, increased exploratory behaviour in the white (increased rearings and line crossings) with corresponding decreased behaviour in the black (decreased rearings and line crossings), which correlated with a reduced % of time spend in the black during the 5 min test session.

The anxiolytic action of AGN 2979 was maintained on continued b.d. treatment. However, in marked contrast to the findings with the diazepam, abrupt cessation of treatment with AGN 2979 was not associated with anxiogenesis. Indeed, in complete contrast, an anxiolytic action was clearly apparent 8h after last dosing with 0.1 mg/kg AGN 2979 and 48 h after last dosing with 10 mg/kg AGN 2979 (Figs. 12 and 13). This anxiolytic action slowly waned over the subsequent 48 h period, and careful observations using separate groups of animals (animals must be naive to the situation) showed that anxiogenesis did not follow the withdrawal of treatment with AGN 2979, and indeed did not develop at any time during a 10 day period following withdrawal of therapy. It is emphasised that this marked contrast in response of mice following withdrawal from subchronic treatments with diazepam and AGN 2979 is apparent when identical treatmen protocols are used.

## EXAMPLE 11

Male laboratory bred common marmosets (Callithrix jacchus), weighing between 350-400g, were housed individually and allowed food (mazuri primate diet S.D.S. Ltd., Essex) 'ad libitum'. Once daily marmosets were also given an assortment of fruit and brown or malt bread. Holding rooms were maintained at 25 ± 1 ° C at a humidity of 55% and on a 12 h light/dark (red illumination) cycle (with simulated dawn and twilight periods) with lights on at 07.00 h.

Tests were conducted between 13.30 - 15.30 h in the normal holding room (to avoid unwanted disruption of behaviour by movement to a novel room or cage). The holding cages, in which marmosets were housed individually for the present work measured 76 cm high, 50 cm wide and 60 cm deep. "Anxiety' was initiated by a human observer standing 0.6 m in front of the holding cage. Changed behaviour was recorded over a 10 minute period (consecutive 2 minute recordings for each behavioural response were measured) both by the observer and by blind assessment by an independent observer of video recordings taken throughout the period of human threat. The behavioural measures selected for the present studies were (a) % of time spent on the cage front in direct confrontation with the human threat and (b) the number of aggressive body postures, primarily shown as raising of the tail to expose the genital region with varying body piloerection, anal scent marking and slit stare with flattened ear tufts.

The results are indicated in Figures 14 and 15 and discussed below.

Figure 14 shows the anxiolytic action of diazepam in the marmoset shown as an increase in the % of time spent on the cage front, and a reduction in the number of postures, when confronted with a human threat standing 0.6 m in front of the holding cage. n = 6. S.E.M.s shown or less than 12.2% *, + P less than 0.001 (one-way ANOVA followed by Dunnett's test).

Figure 15 shows the anxiolytic action of AGN 2979 in the marmoset shown as an increase in the % of time spent on the cage front, and a reduction in the number of postures, when confronted with a human threat standing 0.6 m in front of the holding cage. n = 6. S.E.M.s shown. *, + P less than 0.001 (one-way ANOVA followed by Dunnett's test).

Diazepam was administered subcutaneously at doses of 0.1 and 0.025 mg/kg and AGN 2979 was similarly administered at doses of 0.00001, 0.0001, 0.001 and 1 mg/kg.

Marmosets treated with diazepam exhibited a change in behaviour characteristic of treatment with an anxiolytic agent. Thus, they responded to a human threat by spending more time on the cage front in direct confrontation with the experimenter, and they spent less time directing aggressive postures at the experimenter (shown as a reduction in the number of postures). Diazepam was active to reduce anxiety in the marmoset at doses of 0.01 and 0.025 mg/kg s.c. (Fig.14).

Similarly to diazepam, AGN 2979 was shown to exert anxiolytic activity in the marmoset, seen both as a reduction in the number of postures and an increase in % of time spent on the cage front (Fig. 15). Marmosets treated with AGN 2979 were generally so lacking in fear of the experimenter that they spent considerably more time than control animals jumping backwards and forwards between the cage front and the cage perches, which generally reflects playful behaviour, and this led to an artificial reduction in the % of time spent on the cage front. From the data obtained, there is no indication that AGN 2979 is any less effective than diazepam as an anxiolytic agent in the marmoset AGN 2979 is, however, more potent than diazepam.

The marmoset was found to be particularly sensitive to the sedative actions of diazepam and it was therefore difficult to make behavioural measures on animals treated with doses of diazepam in excess of

0.025 mg/kg. However, sedation was never seen following treatment with AGN 2979.

EXAMPLE 12

The procedure of Example 6 was repeated using 0.01, 0.1, 1 and 10 mg/kg (-)AGN 2979 administered intraperitoncally as a 45 minute pretreatment in a volume of 1ml/100g body weight.

The results are indicated in accompanying Figure 16 and discussed below.

Figure 16 shows changes in rearing behaviour and line crossings (locomotion) in the white and black sections of the box. C indicates the responses of vehicle-treated, control animals. n = 5. S.E.M.s given. Significant increases in responding are indicated as *P less than 0.001, significant decreases as +P less than 0.001 (one-way ANOVA followed by Dunnett's t test).

When given intraperitoneally, using a 45 min pretreatment time, the (-)isomer of AGN 2979 was shown to exert marked anxiolytic activity across the dose range 0.01 - 10 mg/kg. This anxiolytic activity was characterised by increased line crossings and rearing behaviour in the white section of the test box with correspondingly decreased behaviour in the black (Fig.16). The anxiolytic activity of (-)AGN 2979 was also associated with reduced latency for movement from the white to the black section of the test box (normal control values in the range 7 - 12 seconds, this was delayed at all doses of (-)AGN 2979 to 16 - 59 seconds, P less than 0.01 - P less than 0.001), and decreased time spent in the black (normal values in the range 51 - 54%, reduced to 19 - 39% at all doses of (-)AGN 2979, but with the reduction being most marked at the higher doses, P less than 0.001).

The results indicate that (-)AGN 2979 is more potent as an anxiolytic agent than (±)AGN 2979.

**Claims**

**1.** A pharmaceutical composition in unit dose form comprising, with a pharmaceutically acceptable diluent or carrier, a compound of the following Formula I.

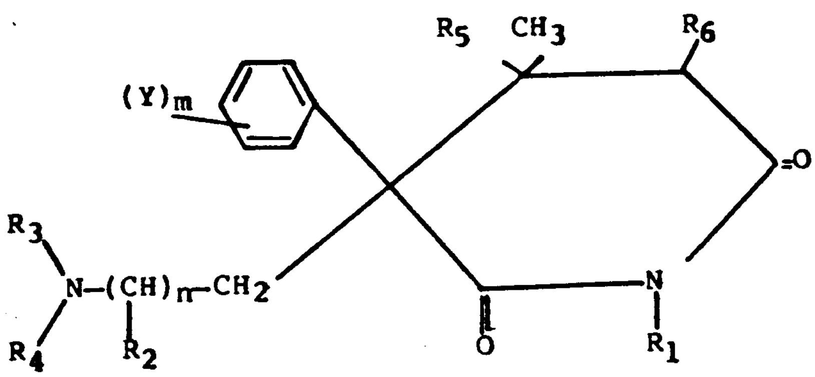

(I)

wherein:

$R_1$ represents hydrogen or $C_1$-$C_4$ alkyl;

n is 1 or 2;

$R_2$ represents hydrogen or methyl, provided that at least one $R_2$ is hydrogen when n is 2;

$R_3$ represents hydrogen or $C_1$-$C_2$ alkyl;

$R_4$ represents $C_1$-$C_2$ alkyl;

$R_5$ and $R_6$ independently represent hydrogen or methyl;

m is 0 to 3; and

each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,

or a pharmacologically acceptable salt thereof, characterized in that the compound of Formula I is present in an amount of $10^{-6}$ to less than $10^{-1}$ mg per unit dose.

**2.** A composition as claimed in Claim 1, wherein the compound has the following Formula IA.

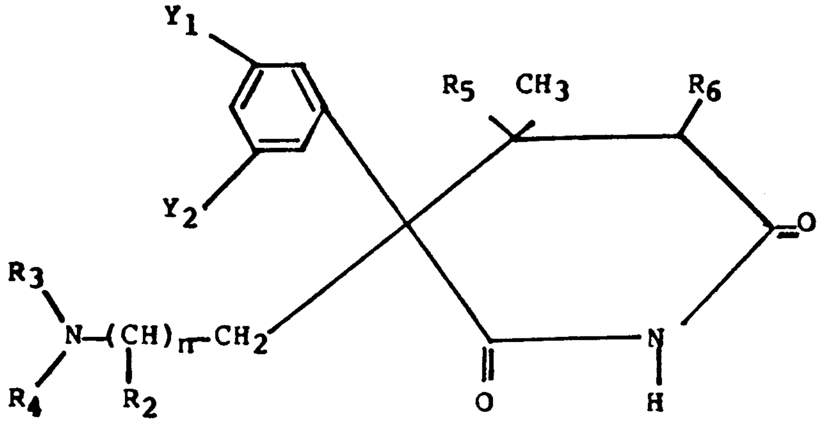

(IA)

wherein:

n is 1 or 2;
$R_2$ represents hydrogen or methyl, provided that at least one $R_2$ is hydrogen when n is 2;
$R_3$ represents hydrogen or $C_1$-$C_2$ alkyl;
$R_4$ represents $C_1$-$C_2$ alkyl;
$R_5$ and $R_6$ independently represent hydrogen or methyl; and
$Y_1$ and $Y_2$ independently represent hydrogen, hydroxy or $C_1$-$C_2$ alkoxy,
or a pharmacologically acceptable salt thereof.

**3.** A composition as claimed in Claim 2, wherein the compound has the following Formula IB.

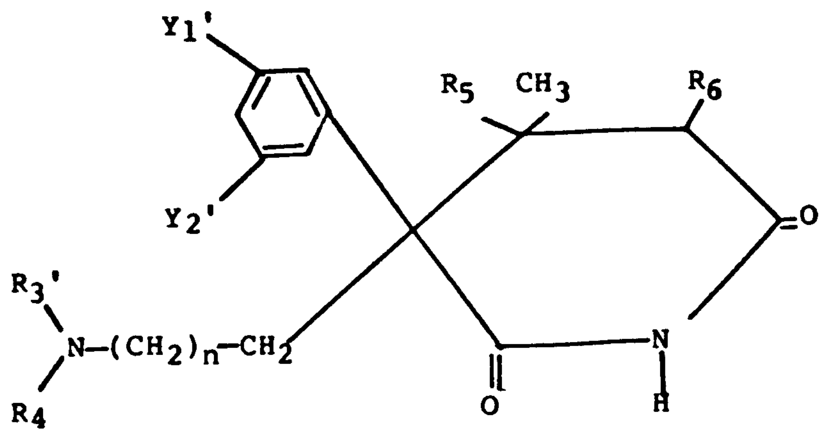

(IB)

wherein:

n is 1 or 2;
$R_3$' and $R_4$ independently represent $C_1$-$C_2$ alkyl;
$R_5$ and $R_6$ independently represent hydrogen or methyl; and
$Y_1$' and $Y_2$' independently represent hydrogen, hydroxy or $C_1$-$C_2$ alkoxy,
or a pharmacologically acceptable salt thereof.

**4.** A composition as claimed in Claim 3, wherein $Y_1$' represents hydroxy or methoxy and $Y_2$' represents hydrogen, hydroxy or methoxy.

**5.** A composition as claimed in Claim 3, wherein the compound is 3-(3'-methoxyphenyl)-3-(3"-N,N-dimethylaminopropyl)-4,4-dimethyl-2,6-dioxopiperidine.

**6.** A composition as claimed in Claim 5 wherein the compound is the minus isomer.

**7.** A composition as claimed in Claim 3, wherein the compound is 3-(3'hydroxyphenyl)-3-(3"-N,N-dimethylaminopropyl)-4,4-dimethyl-2,6-dioxopiperidine.

**8.** A composition as claimed in any one of the preceding claims which contains $10^{-5}$ to less than $10^{-1}$ mg of the said compound of Formula I.

**9.** The use as an anxiolytic agent in an amount of $10^{-6}$ to $10^{-1}$ mg per unit dose in the manufacture of a medicament for the treatment of anxiety of a compound of the following Formula I.

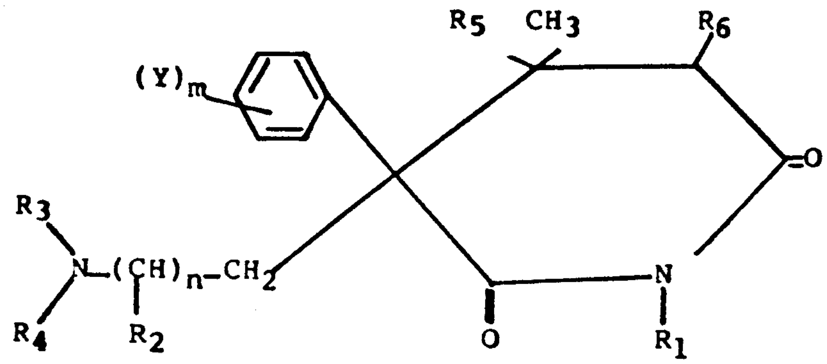

(I)

wherein:

$R_1$ represents hydrogen or $C_1$-$C_4$ alkyl;
$\underline{n}$ is 1 or 2;
$R_2$ represents hydrogen or methyl, provided that at least one $R_2$ is hydrogen when $\underline{n}$ is 2;
$R_3$ represents hydrogen or $C_1$-$C_2$ alkyl;
$R_4$ represents $C_1$-$C_2$ alkyl;
$R_5$ and $R_6$ independently represent hydrogen or methyl;
$\underline{m}$ is 0 to 3; and
each Y is in a meta or para position and independently represents hydroxy, $C_1$-$C_2$ alkoxy, $C_1$-$C_2$ alkyl, $C_1$-$C_2$ hydroxyalkyl, halogen, or trifluoromethyl, provided that hydroxy and alkoxy are not in the para position,
or a pharmacologically acceptable salt thereof.

**10.** A use as claimed in Claim 9, wherein the compound has the following Formula IA.

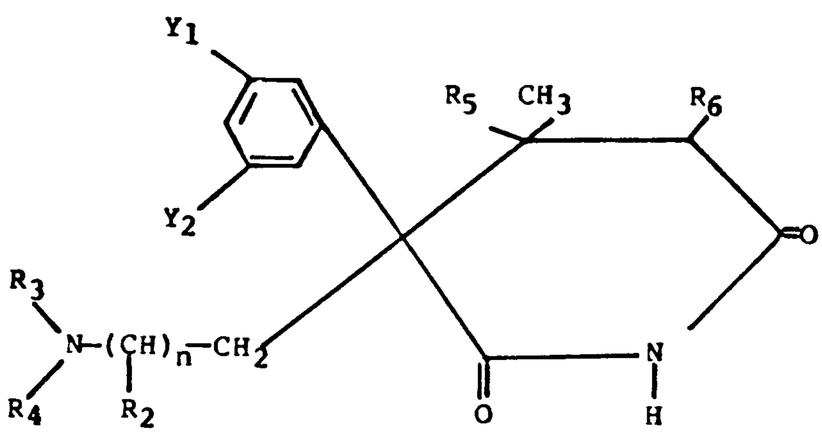

(IA)

wherein:

$\underline{n}$ is 1 or 2;
$R_2$ represents hydrogen or methyl, provided that at least one $R_2$ is hydrogen when $\underline{n}$ is 2;
$R_3$ represents hydrogen or $C_1$-$C_2$ alkyl;
$R_4$ represents $C_1$-$C_2$ alkyl;
$R_5$ and $R_6$ independently represent hydrogen or methyl; and
$Y_1$ and $Y_2$ independently represent hydrogen, hydroxy or $C_1$-$C_2$ alkoxy,
or a pharmacologically acceptable salt thereof.

**11.** A use as claimed in Claim 10, wherein the compound has the following Formula IB.

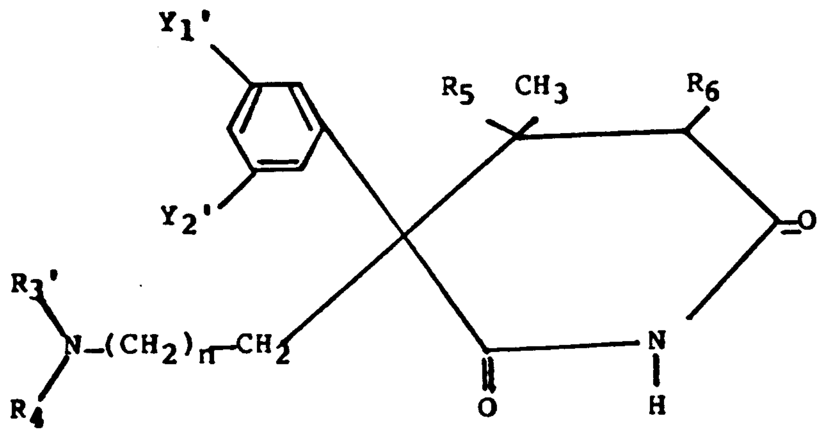

(IB)

wherein:

n is 1 or 2;

$R_3$' and $R_4$ independently represent $C_1$-$C_2$ alkyl;

$R_5$ and $R_6$ independently represent hydrogen or methyl;

$Y_1$' represents hydroxy or $C_1$-$C_2$ alkoxy; and

$Y_{2'}$ represents hydrogen, hydroxy or $C_1$-$C_2$ alkoxy,

or a pharmacologically acceptable salt thereof.

**12.** A use as claimed in Claim 11, wherein $Y_1$' represents hydroxy or methoxy and $Y_{2'}$ represents hydrogen, hydroxy or methoxy.

**13.** A use as claimed in Claim 11, wherein the compound is 3-(3'-methoxyphenyl)-3-(3"-N,N-dimethylaminopropyl)-4,4-dimethyl-2,6-dioxopiperidine.

**14.** A use as claimed in Claim 13, wherein the compound is the minus isomer.

**15.** A use as claimed in Claim 11, wherein the compound is 3-(3'hydroxyphenyl)-3-(3"-N,N-dimethylaminopropyl)-4,4-dimethyl-2,6-dioxopiperidine.

**16.** A use as claimed in any one of Claims 9 to 15, wherein the medicament contains $10^{-4}$ to $10^{-1}$ mg per unit dose of the said compound of Formula I.

## Revendications

**1.** Composition pharmaceutique sous forme de dose unitaire comprenant, avec un diluant ou un véhicule pharmaceutiquement acceptable, un composé de la formule I suivante :

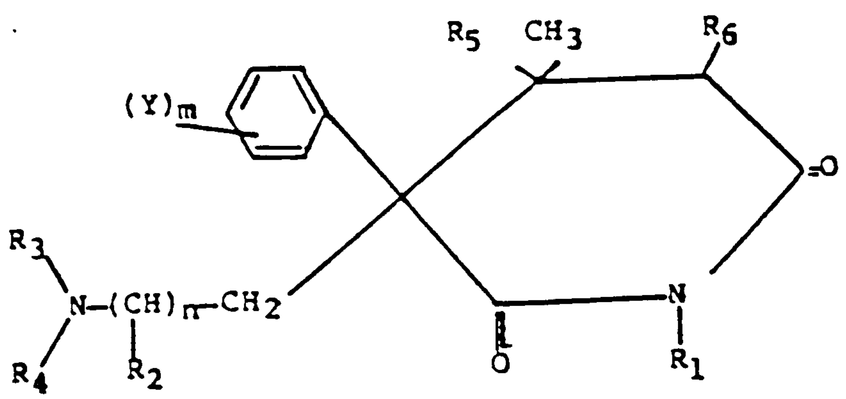

(I)

dans laquelle :
$R_1$ représente un hydrogène ou un alcoyle à 1 à 4 atomes de carbone;
n = 1 ou 2;
$R_2$ représente un hydrogène ou un groupe méthyle, pourvu qu'au moins l'un des $R_2$ soit un hydrogène lorsque n = 2;
$R_3$ représente un hydrogène ou un alcoyle à 1 à 2 atomes de carbone;
$R_4$ représente un alcoyle à 1 à 2 atomes de carbone;
$R_5$ et $R_6$ représentent indépendamment un hydrogène ou un groupe méthyle;
m = 0, 1, 2 ou 3; et
chaque Y est dans une position méta ou para et représente indépendamment un hydroxy, un alcoxy à 1 à 2 atomes de carbone, un alcoyle à 1 à 2 atomes de carbone, un hydroxyalcoyle à 1 à 2 atomes de carbone, un halogène ou le trifluorométhyle, pourvu qu'hydroxy et alcoxy ne soient pas dans la position para,
ou un de leurs sels pharmacologiquement acceptables, caractérisé en ce que le composé de la formule I est présent en une quantité de $10^{-6}$ à moins de $10^{-1}$ mg/dose unitaire.

**2.** Composition selon la revendication 1, dans laquelle le composé a la formule IA suivante :

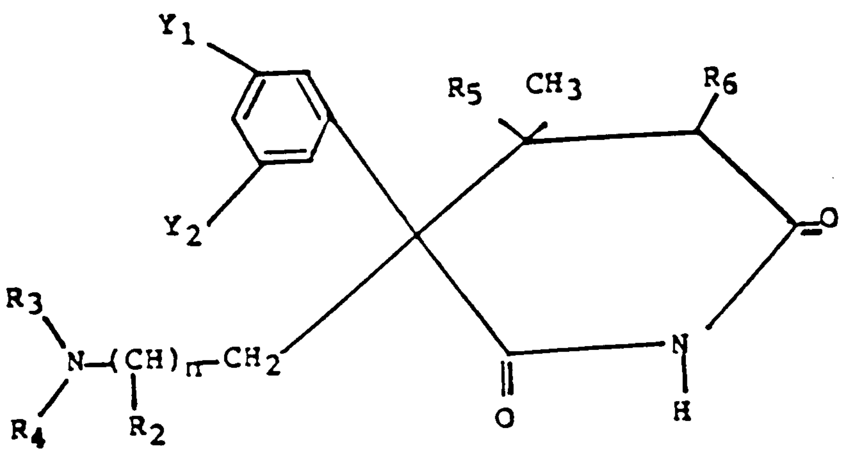

(IA)

dans laquelle :
n est égal à 1 ou 2;
$R_2$ représente un hydrogène ou un groupe méthyle, pourvu qu'au moins l'un des $R_2$ soit un hydrogène lorsque n = 2;
$R_3$ représente un hydrogène ou un alcoyle à 1 ou 2 atomes de carbone;
$R_4$ représente un alcoyle à 1 à 2 atomes de carbone;
$R_5$ et $R_6$ représentent indépendamment un hydrogène ou un groupe méthyle; et
$Y_1$ et $Y_2$ représentent indépendamment un hydrogène, un hydroxy ou un alcoxy à 1 à 2 atomes de carbone,
ou un de leurs sels pharmacologiquement acceptables.

**3.** Composition selon la revendication 2, dans laquelle le composé a la formule IB suivante :

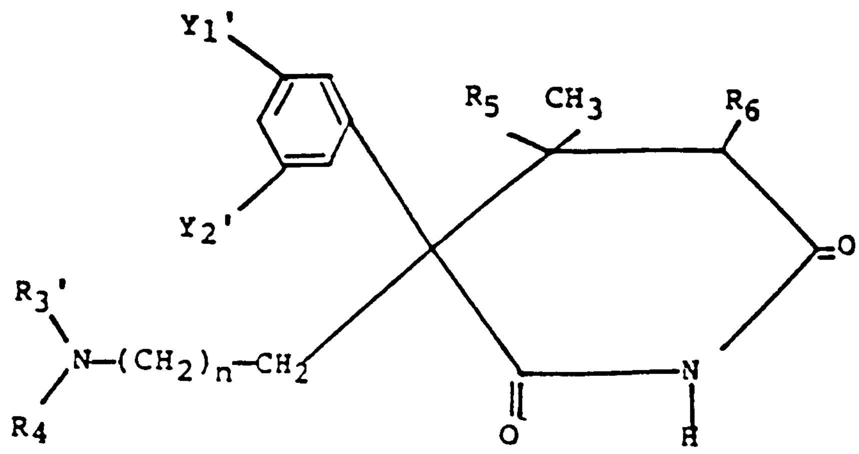

(IB)

dans laquelle :
n est égal à 1 ou 2;
$R'_3$ et $R_4$ représentent indépendamment un alcoyle à 1 à 2 atomes de carbone;
$R_5$ et $R_6$ représentent indépendamment un hydrogène ou un groupe méthyle; et
$Y_1'$ et $Y_2'$ représentent indépendamment un hydrogène, un hydroxy ou un alcoxy à 1 à 2 atomes de carbone,
ou un de leurs sels pharmacologiquement acceptables.

4. Composition selon la revendication 3, dans laquelle $Y_1'$ représente un hydroxy ou un éthoxy et $Y_2'$ représente un hydrogène, un hydroxy ou un méthoxy.

5. Composition selon la revendication 3, dans laquelle le composé est 3(3'-méthoxyphényl)-3-(3"-N,N-diméthylaminopropyl)-4,4-diméthyl-2,6-dioxopipéridine.

6. Composition selon la revendication 5, dans laquelle le composé est l'isomère moins.

7. Composition selon la revendication 3, dans laquelle le composé est 3-(3'-hydroxyphényl)-3-(3"-N,N-diméthylaminopropyl)-4,4-diméthyl-2,6-dioxopipéridine.

8. Composition selon l'une des revendications précédentes, qui contient $10^{-5}$ à moins de 10-1 mg du composé de la formule I.

9. Utilisation comme agent anxiolytique en une quantité de $10^{-6}$ à $10^{-1}$ mg/dose unitaire dans la préparation d'un médicament pour le traitement de l'anxiété d'un composé de la formule I suivante :

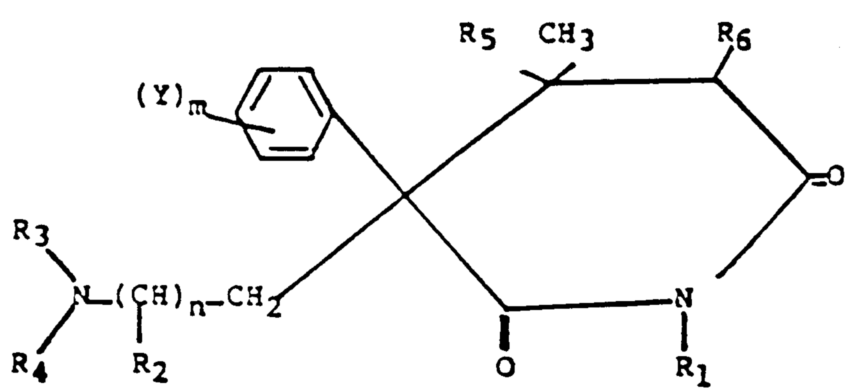

(I)

dans laquelle :
$R_1$ représente un hydrogène ou un alcoyle à 1 à 4 atomes de carbone;
n est égal à 1 ou 2;

$R_2$ représente un hydrogène ou un groupe méthyle, pourvu qu'au moins l'un des $R_2$ soit un hydrogène lorsque n est égal à 2;
$R_3$ représente un hydrogène ou un alcoyle à 1 à 2 atomes de carbone;
$R_4$ représente un alcoyle à 1 à 2 atomes de carbone;
$R_5$ et $R_6$ représentent indépendamment un hydrogène ou un groupe méthyle;
m est égal à 0, 1, 2 ou 3; et
chaque Y est dans une position méta ou para et représente indépendamment un hydroxy, un alcoxy à 1 à 2 atomes de carbone, un alcoyle à 1 à 2 atomes de carbone, un hydroxyalcoyle à 1 à 2 atomes de carbone, un halogène ou le trifluorométhyle, pourvu que hydroxy et alcoxy ne soient pas dans la position para;
ou un de leurs sels pharmacologiquement acceptables.

**10.** Utilisation selon la revendication 9, dans laquelle le composé a la formule IA suivante :

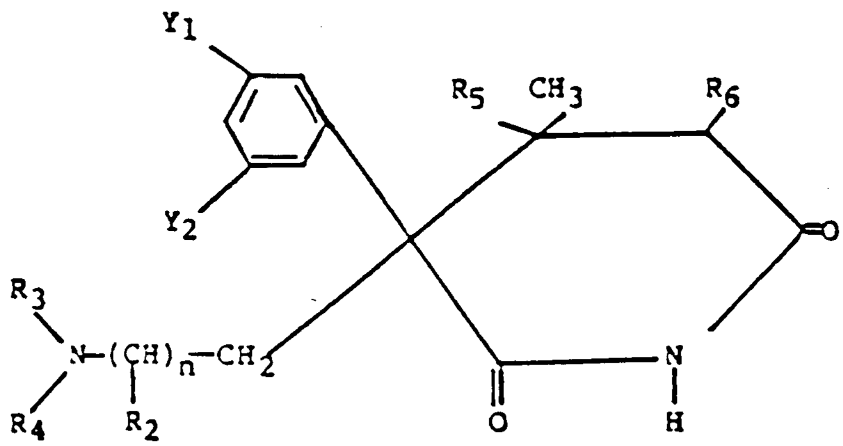

(IA)

dans laquelle :
n est égal à 1 ou 2;
$R_2$ représente un hydrogène ou un groupe méthyle, pourvu qu'au moins l'un des $R_2$ soit de l'hydrogène lorsque n est égal à 2;
$R_3$ représente un hydrogène ou un alcoyle à 1 à 2 atomes de carbone;
$R_4$ représente un alcoyle à 1 ou 2 atomes de carbone;
$R_5$ et $R_6$ représentent indépendamment un hydrogène ou un groupe méthyle; et
$Y_1$ et $Y_2$ représentent indépendamment un hydrogène, un hydroxy ou un alcoxy à 1 à 2 atomes de carbone;
ou un de leurs sels pharmacologiquement acceptables.

**11.** Utilisation selon la revendication 10, dans laquelle le composé a la formule IB suivante :

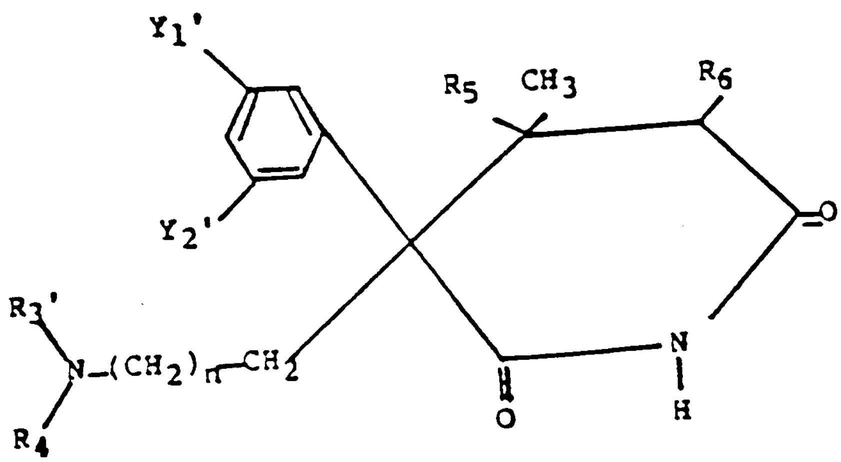

(IB)

dans laquelle :
n est égal à 1 ou 2;
$R'_3$ et $R_4$ représentent indépendamment un alcoyle à 1 à 2 atomes de carbone;
$R_5$ et $R_6$ représentent indépendamment un hydrogène ou un groupe méthyle;
$Y_1$' représente un hydroxy ou un alcoxy à 1 ou 2 atomes de carbone; et
$Y_2$' représente un hydrogène, un hydroxy ou un alcoxy à 1 ou 2 atomes de carbone;
ou un de leurs sels pharmacologiquement acceptables.

**12.** Utilisation selon la revendication 11, dans laquelle $Y_1$' représente un hydroxy ou un méthoxy et $Y_2$' représente un hydrogène, un hydroxy ou un méthoxy.

**13.** Utilisation selon la revendication 11, dans laquelle le composé est 3(3'-méthoxyphényl)-3-(3'-N,N-diméthylaminopropyl)-4,4-diméthyl-2,6-dioxopipéridine.

**14.** Utilisation selon la revendication 13, dans laquelle le composé est l'isomère moins.

**15.** Utilisation selon la revendication 11, dans laquelle le composé est 3-(3'-hydroxyphényl)-3-(3"-N,N-diméthylaminopropyl)-4,4-diméthyl-2,6-dioxopipéridine.

**16.** Utilisation selon l'une des revendications 9 à 15, dans laquelle le médicament contient $10^{-4}$ à $10^{-1}$ mg/dose unitaire du composé de la formule I.

**Patentansprüche**

**1.** Pharmazeutische Zusammensetzung in Einzeldosisform enthaltend zusammen mit einem pharmazeutisch annehmbaren Verdünnungsmittel oder Träger eine Verbindung entsprechend der nachstehenden allgemeinen Formel (I)

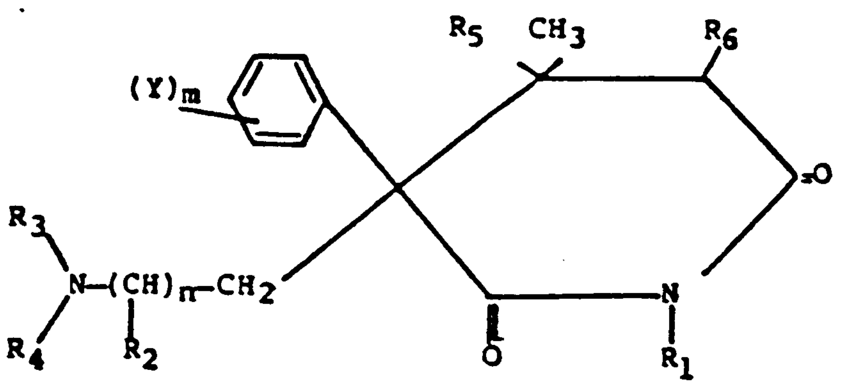

(I)

in der

| | |
|---|---|
| $R_1$ | Wasserstoff oder eine $C_1$-$C_4$-Alkylgruppe ist, |
| n | 1 oder 2 bedeutet, |
| $R_2$ | Wasserstoff oder Methyl entspricht, aber dabei im Falle von n = 2 wenigstens ein $R_2$ Wasserstoff ist, |
| $R_3$ | Wasserstoff oder eine $C_1$-$C_2$-Alkylgruppe darstellt, |
| $R_4$ | eine $C_1$-$C_2$-Alkylgruppe bedeutet, |
| $R_5$ und $R_6$ | unabhängig voneinander Wasserstoff oder Methyl entsprechen, |
| m | einen Wert von 0 bis 3 hat und |

jedes Y in meta oder para-Stellung steht und unabhängig voneinander Hydroxy, $C_1$-$C_2$-Alkoxy, $C_1$-$C_2$-Alkyl, $C_1$-$C_2$-Hydroxyalkyl, Halogen oder Trifluormethyl ist, jedoch Hydroxy und Alkoxy sich nicht in Parastellung befinden,
oder ein pharmakologisch annehmbares Salz davon,
dadurch **gekennzeichnet,**
daß die Verbindung entsprechend der allgemeinen Formel (I) in einer Menge von $10^{-6}$ bis zu weniger als $10^{-1}$ mg je Einzeldosis vorliegt.

2. Zusammensetzung gemäß Anspruch 1, wobei die Verbindung der nachstehenden allgemeinen Formel (IA) entspricht:

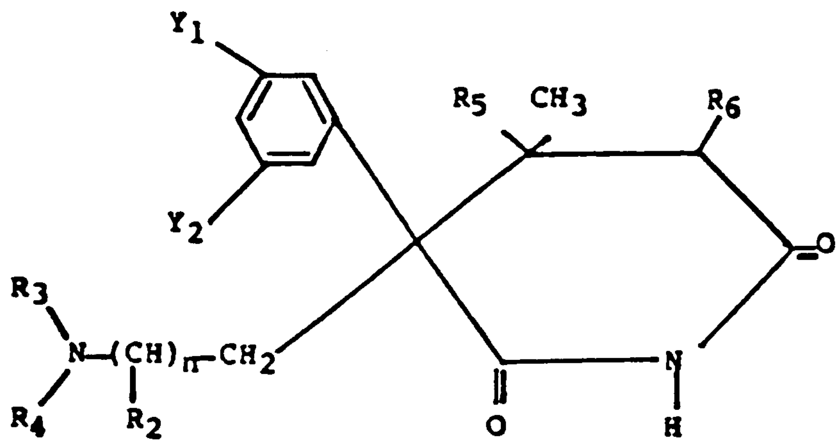

(IA)

in der

| | |
|---|---|
| n | 1 oder 2 ist, |
| $R_2$ | Wasserstoff oder Methyl entspricht, jedoch im Falle von n = 2 wenigstens ein $R_2$ Wasserstoff ist, |
| $R_3$ | Wasserstoff oder eine $C_1$-$C_2$-Alkylgruppe darstellt, |
| $R_4$ | eine $C_1$-$C_2$-Alkylgruppe entspricht, |
| $R_5$ und $R_6$ | unabhängig voneinander Wasserstoff oder Methyl bedeuten und |
| $Y_1$ und $Y_2$ | unabhängig voneinander Wasserstoff, Hydroxy oder $C_1$-$C_2$-Alkoxygruppen sind, |

oder ein pharmakologisch annehmbares Salz davon ist.

3. Zusammensetzung gemäß Anspruch 2, wobei die Verbindung der nachfolgenden allgemeinen Formel (IB) entspricht;

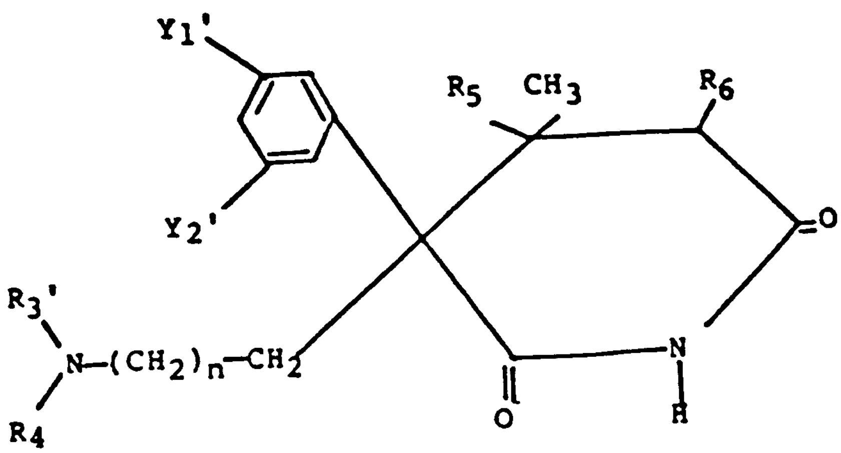

(IB)

in der

| | |
|---|---|
| n | 1 oder 2 ist, |
| R3' und $R_4$ | unabhängig voneinander $C_1$-$C_2$-Alkylgruppen sind, |
| $R_5$ und $R_6$ | unabhängig voneinander Wasserstoff oder Methyl bedeuten und |
| $Y_1$' und $Y_2$' | unabhängig voneinander Wasserstoff, Hydroxy oder $C_1$-$C_2$-Alkoxygruppen sind, |

oder ein pharmakologisch annehmbares Salz davon ist.

4. Zusammensetzung gemäß Anspruch 3, wobei $Y_1$' Hydroxy oder Methoxy ist und $Y_2$' Wasserstoff, Hydroxy oder Methoxy entspricht.

**5.** Zusammensetzung gemäß Anspruch 3, wobei die Verbindung 3-(3'-Methoxyphenyl)-3-(3"-N,N-dimethylaminopropyl)-4,4-dimethyl-2,6-dioxopiperidin ist.

**6.** Zusammensetzung gemäß Anspruch 5, wobei die Verbindung das Minus-Isomere ist.

**7.** Zusammensetzung gemäß Anspruch 3, wobei die Verbindung 3-(3'-Hydroxyphenyl)-3-(3"-N,N-dimethylaminopropyl)-4,4-dimethyl-2,6-dioxopiperidin ist.

**8.** Zusammensetzung gemäß irgendeinem der vorangehenden Ansprüche, enthaltend $10^{-5}$ bis weniger als $10^{-1}$ mg von der Verbindung entsprechend der allgemeinen Formel (I).

**9.** Verwendung einer Verbindung entsprechend der nachstehenden allgemeinen Formel (I)

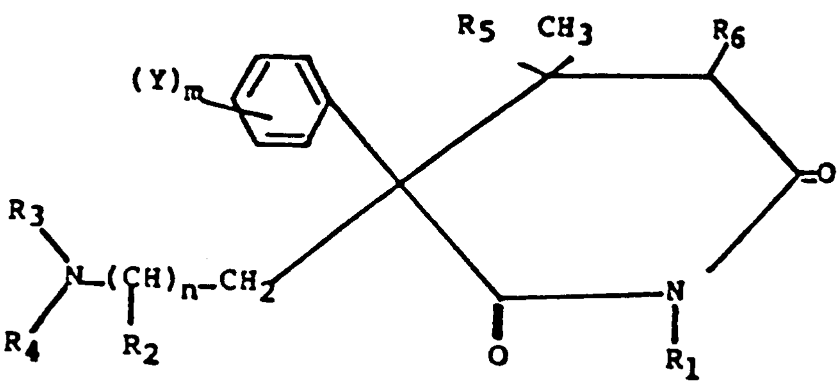

(I)

in der

| | |
|---|---|
| $R_1$ | Wasserstoff oder eine $C_1$-$C_4$-Alkylgruppe ist, |
| n | 1 oder 2 bedeutet, |
| $R_2$ | Wasserstoff oder Methyl entspricht, aber dabei im Falle von n = 2 wenigstens ein $R_2$ Wasserstoff ist, |
| $R_3$ | Wasserstoff oder eine $C_1$-$C_2$-Alkylgruppe darstellt, |
| $R_4$ | eine $C_1$-$C_2$-Alkylgruppe bedeutet, |
| $R_5$ und $R_6$ | unabhängig voneinander Wasserstoff oder Methyl entsprechen, |
| m | einen Wert von 0 bis 3 hat und |

jedes Y in meta oder para-Stellung steht und unabhängig voneinander Hydroxy, $C_1$-$C_2$-Alkoxy, $C_1$-$C_2$-Alkyl, $C_1$-$C_2$-Hydroxyalkyl, Halogen oder Trifluormethyl ist, jedoch Hydroxy und Alkoxy sich nicht in Parastellung befinden,
oder eines pharmakologisch annehmbares Salzes davon,
als Anxiolytikum in einer Menge von $10^{-6}$ bis $10^{-1}$ mg je Einzeldosis bei der Herstellung eines Medikaments zur Behandlung von Angstzusränden.

**10.** Verwendung gemäß Anspruch 9, wobei die Verbindung der nachstehenden allgemeinen Formel (IA) entspricht

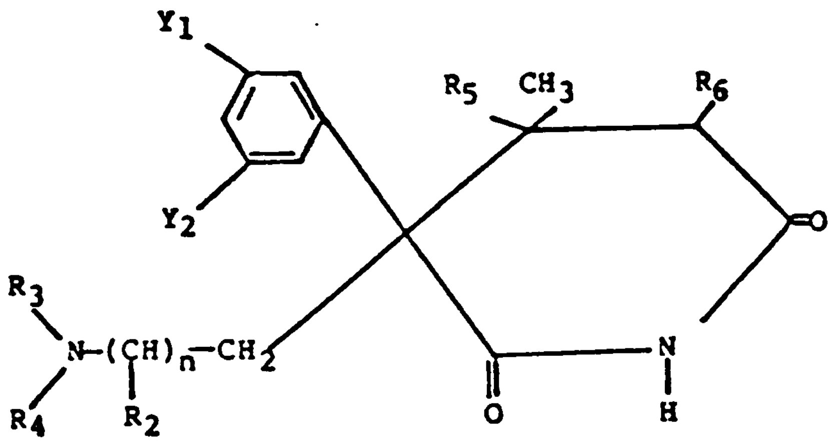

(IA)

in der

n 1 oder 2 ist,

$R_2$ Wasserstoff oder Methyl entspricht, jedoch im Falle von n = 2 wenigstens ein $R_2$ Wasserstoff ist,

$R_3$ Wasserstoff oder eine $C_1$-$C_2$-Alkylgruppe darstellt,

$R_4$ eine $C_1$-$C_2$-Alkylgruppe entspricht,

$R_5$ und $R_6$ unabhängig voneinander Wasserstoff oder Methyl bedeuten und

$Y_1$ und $Y_2$ unabhängig voneinander Wasserstoff, Hydroxy oder $C_1$-$C_2$-Alkoxygruppen sind,

oder ein pharmakologisch annehmbares Salz davon ist.

**11.** Verwendung gemäß Anspruch 10, wobei die Verbindung der nachstehenden allgemeinen Formel (IB) entspricht

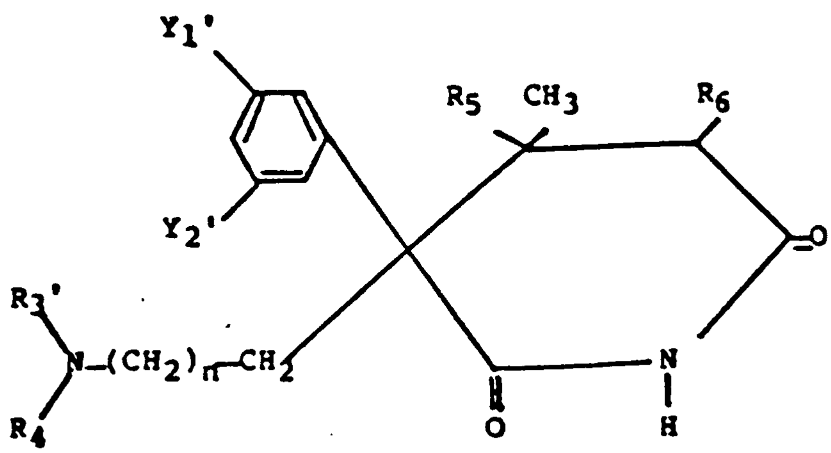

(IB)

in der

n 1 oder 2 ist,

$R_3$' und $R_4$ unabhängig voneinander $C_1$-$C_2$-Alkylgruppen sind,

$R_5$ und $R_6$ unabhängig voneinander Wasserstoff oder Methyl bedeuten,

$Y_1$' Hydroxy oder eine $C_1$-$C_2$-Alkoxygruppe darstellt und

$Y_2$' Wasserstoff Hydroxy oder eine $C_1$-$C_2$-Alkoxygruppe bedeutet,

oder ein pharmakologisch annehmbares Salz davon ist.

**12.** Verwendung gemäß Anspruch 11, wobei $Y_1$' Hydroxy oder Methoxy ist und $Y_2$' Wasserstoff, Hydroxy oder Methoxy entspricht.

**13.** Verwendung gemäß Anspruch 11, wobei die Verbindung 3-(3'-Methoxyphenyl)-3-(3"-N,N-dimethylaminopropyl)-4,4-dimethyl-2,6-dioxopiperidin ist.

**14.** Verwendung gemäß Anspruch 13, wobei die Verbindung das Minus-Isomere ist.

**15.** Verwendung gemäß Anspruch 11, wobei die Verbindung 3-(3'-Hydroxyphenyl)-3-(3"-N,N-dlmethylamlnopropyl)-4,4-dimethyl-2,6-dioxopiperidin ist.

**16.** Verwendung gemäß irgendeinem der Ansprüche 9 bis 15, wobei das Medikament je Einzeldosis $10^{-4}$ bis $10^{-1}$ mg von der Verbindung mit der Formel (I) enthält.

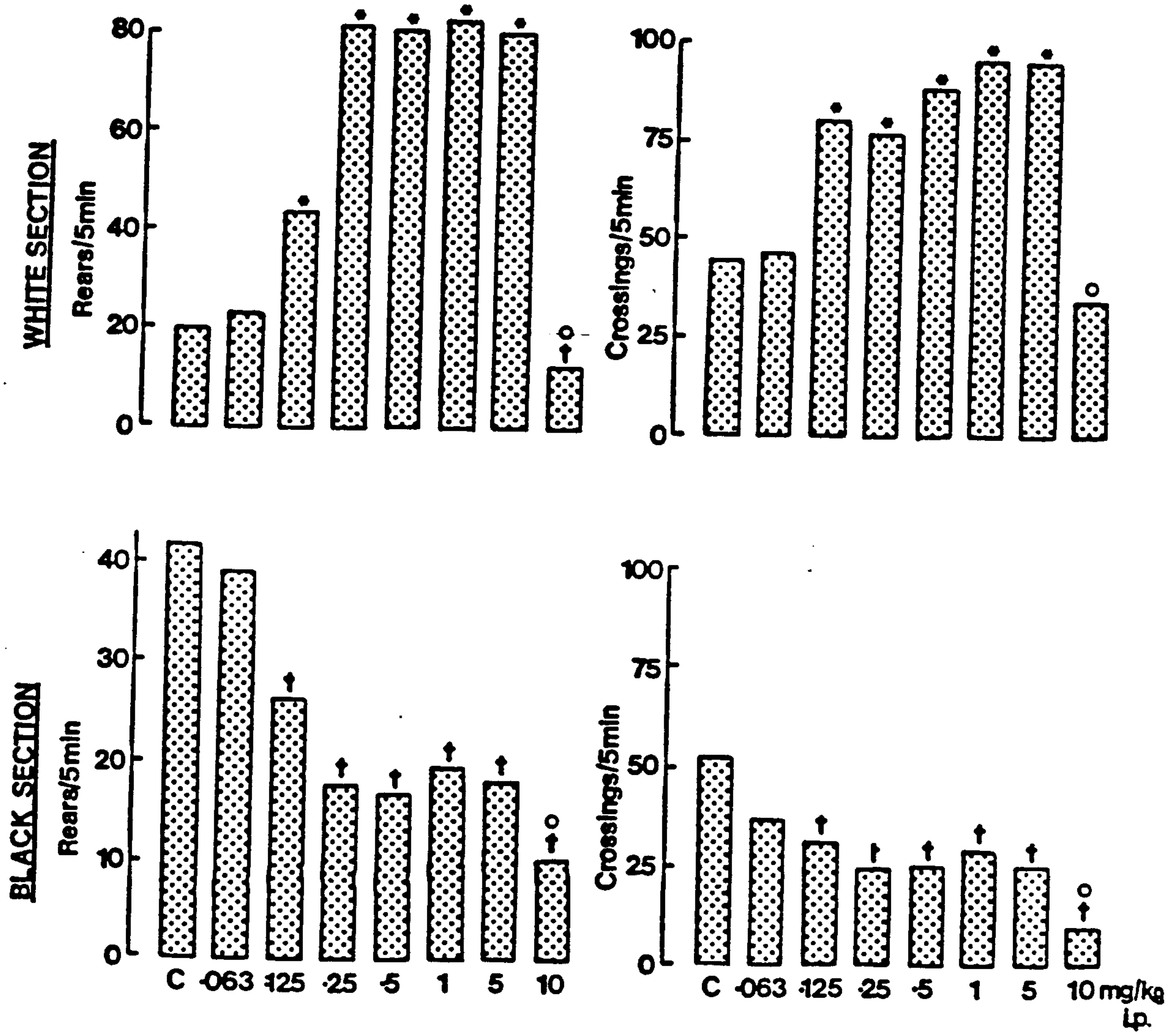
WHITE SECTION
Rears/5min
Crossings/5min
BLACK SECTION
Rears/5min
Crossings/5min
C ·063 ·125 ·25 ·5 1 5 10
C ·063 ·125 ·25 ·5 1 5 10 mg/kg
i.p.

FIGURE 1

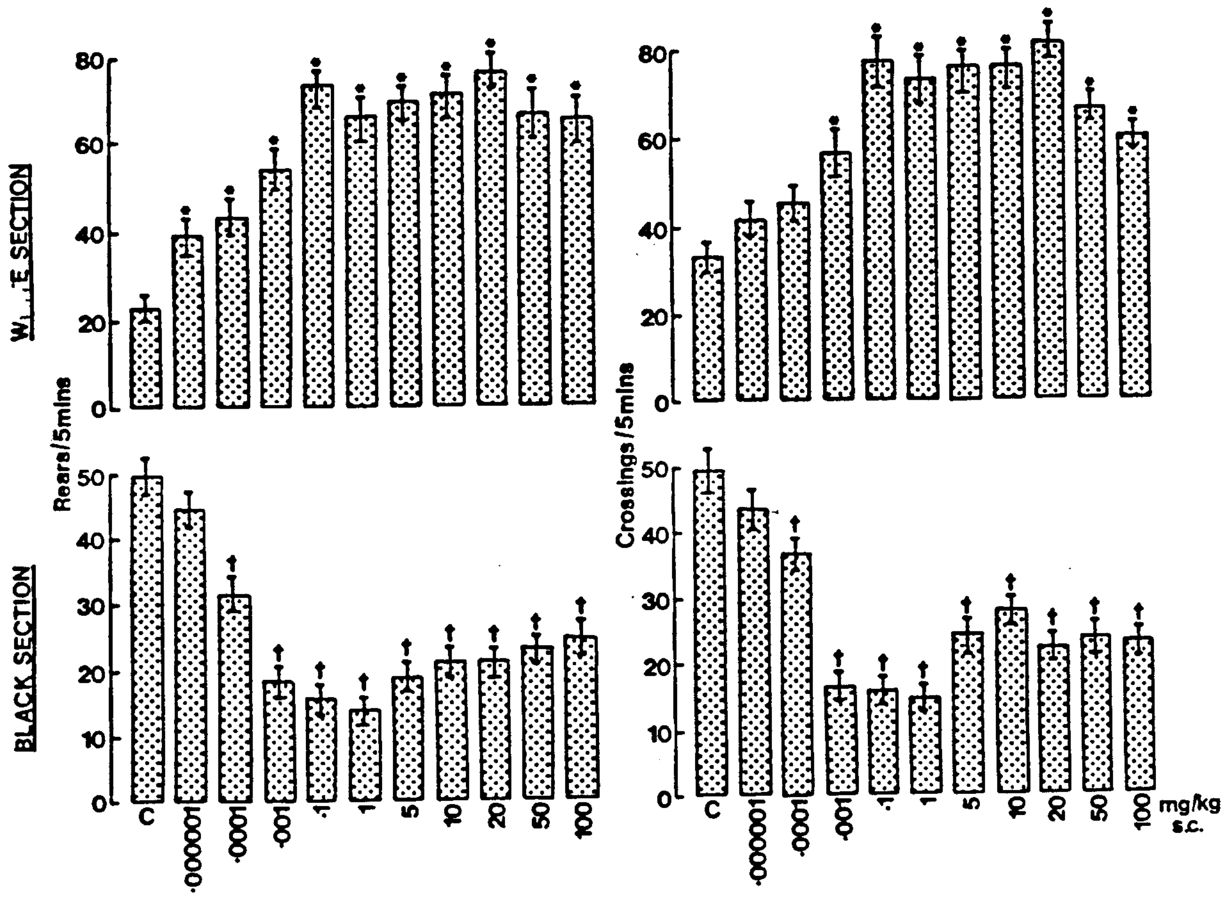

W... E SECTION
BLACK SECTION
Rears/5mins
Crossings/5mins
80
60
40
20
0
50
40
30
20
10
C
·000001
·0001
·001
·1
1
5
10
20
50
100
mg/kg
s.c.


FIGURE 2

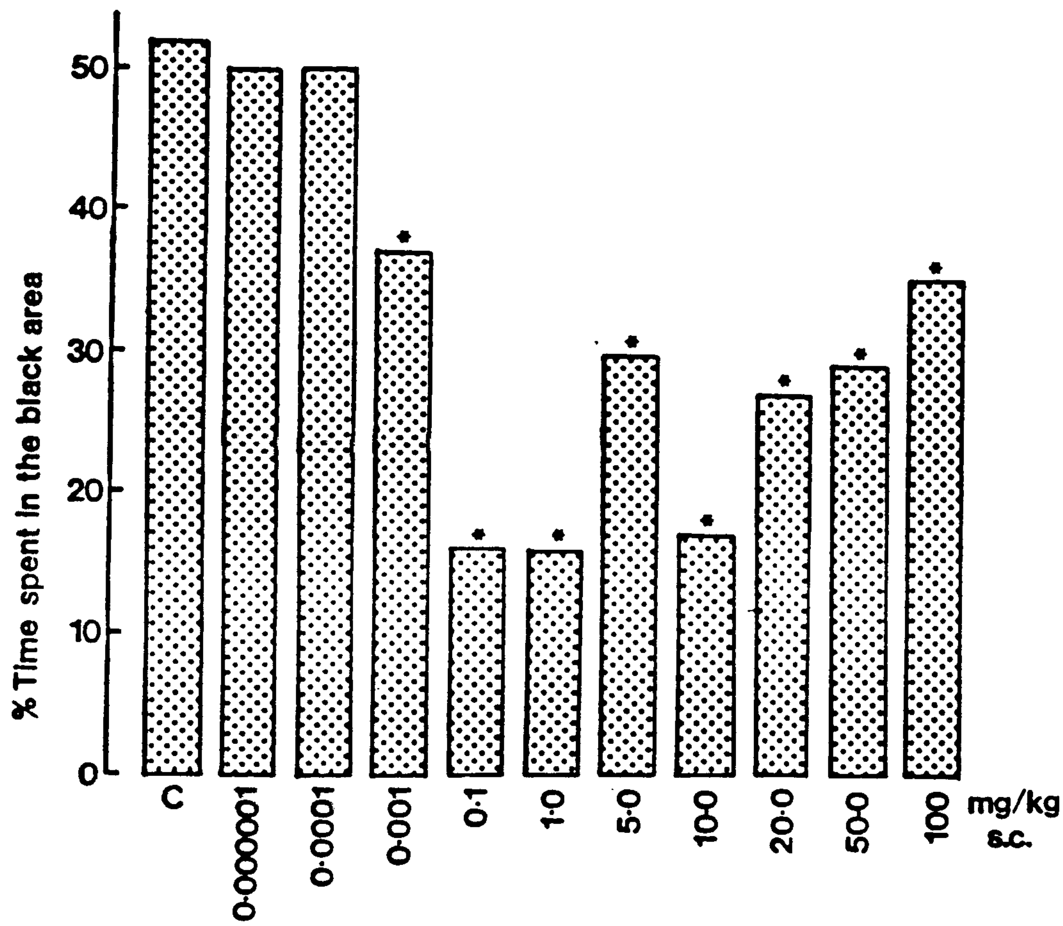
% Time spent in the black area
0
10
20
30
40
50
C
0·000001
0·0001
0·001
0·1
1·0
5·0
10·0
20·0
50·0
100
mg/kg
s.c.

FIGURE 3

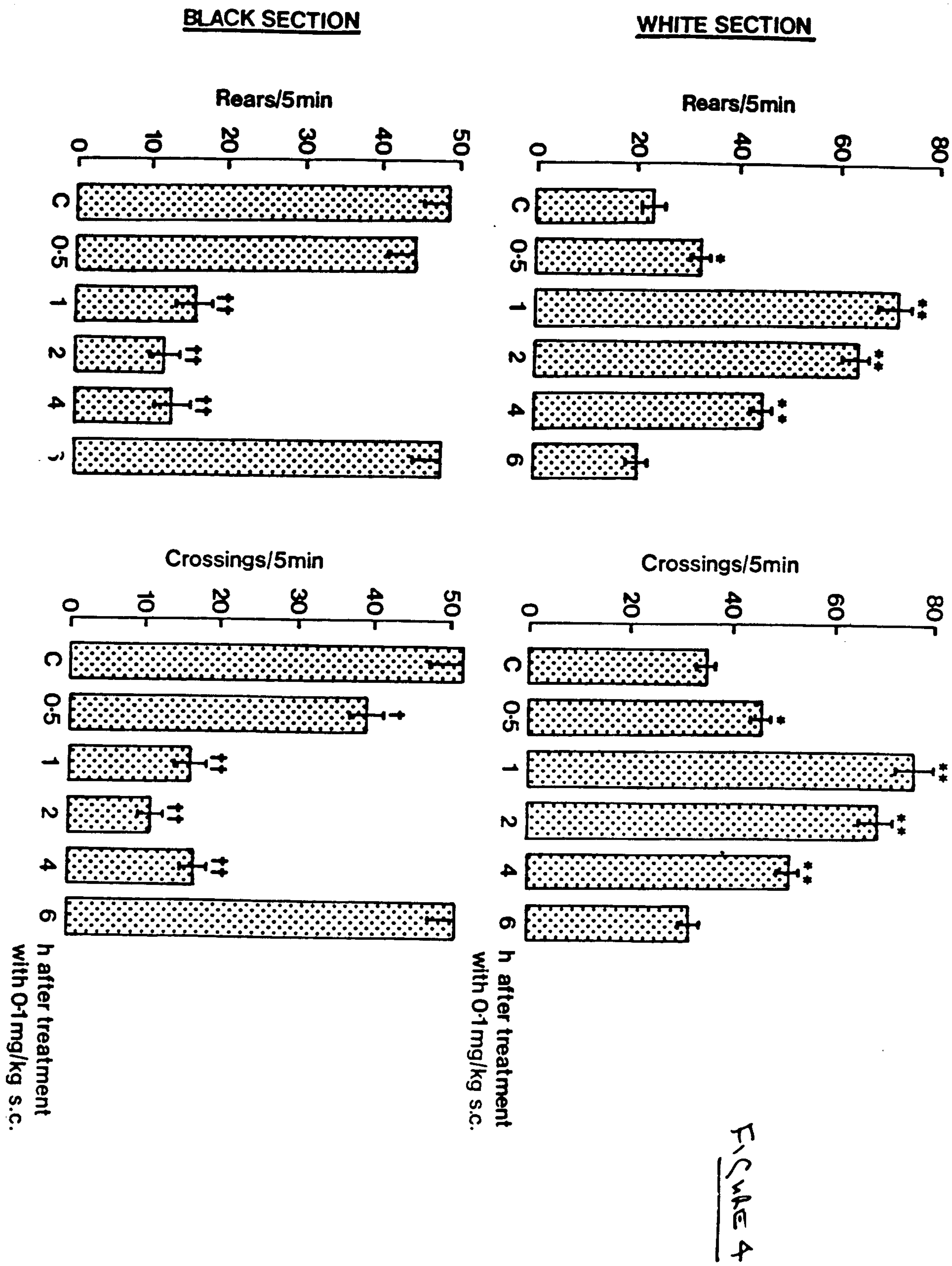

BLACK SECTION
WHITE SECTION
Rears/5min
0 10 20 30 40 50
C 0·5 1 2 4 6
Rears/5min
0 20 40 60 80
C 0·5 1 2 4 6
Crossings/5min
0 10 20 30 40 50
C 0·5 1 2 4 6
h after treatment with 0·1mg/kg s.c.
Crossings/5min
0 20 40 60 80
C 0·5 1 2 4 6
h after treatment with 0·1mg/kg s.c.


FIGURE 4

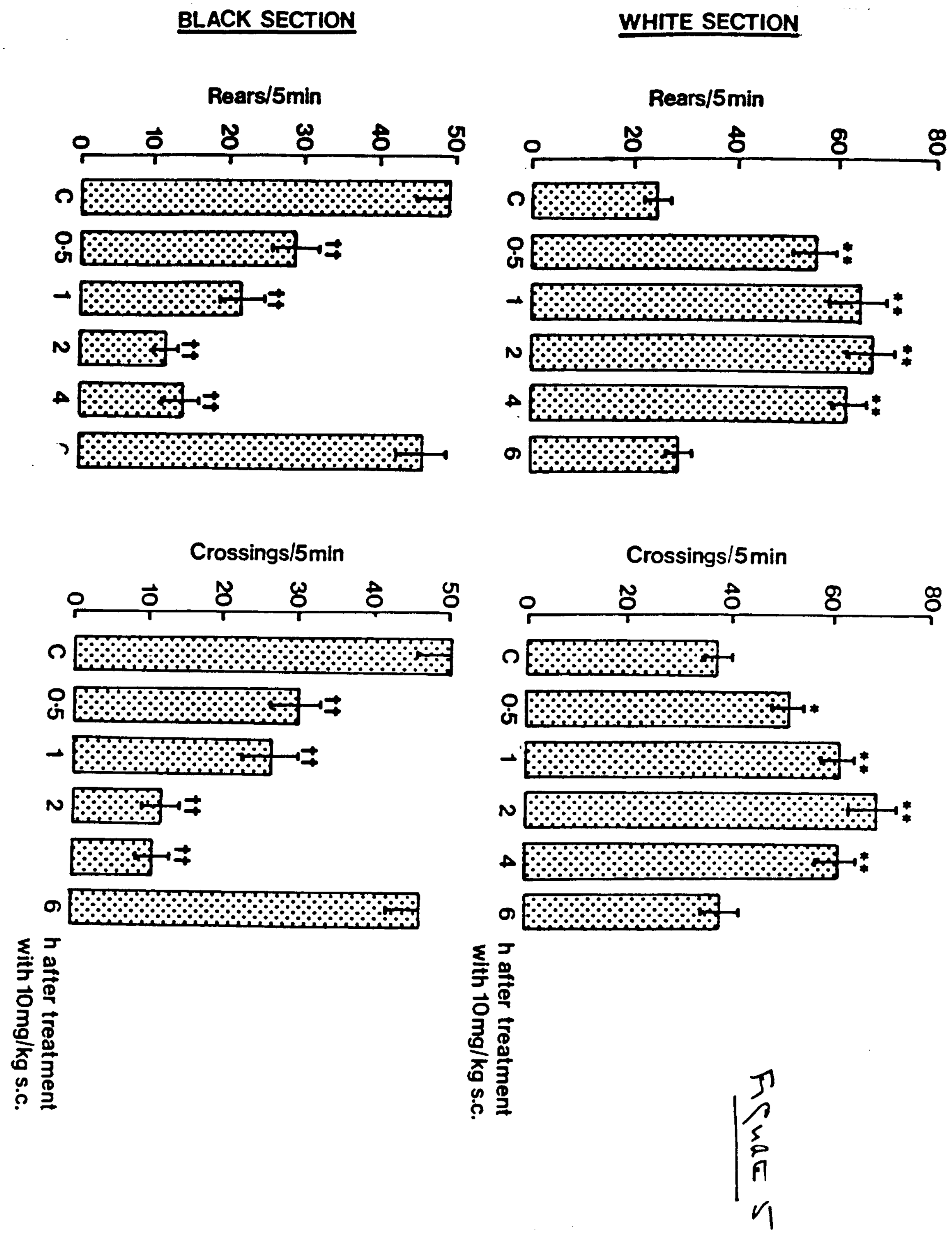
BLACK SECTION
WHITE SECTION
Rears/5min
Rears/5min
Crossings/5min
Crossings/5min
h after treatment with 10mg/kg s.c.
h after treatment with 10mg/kg s.c.
Figure 5

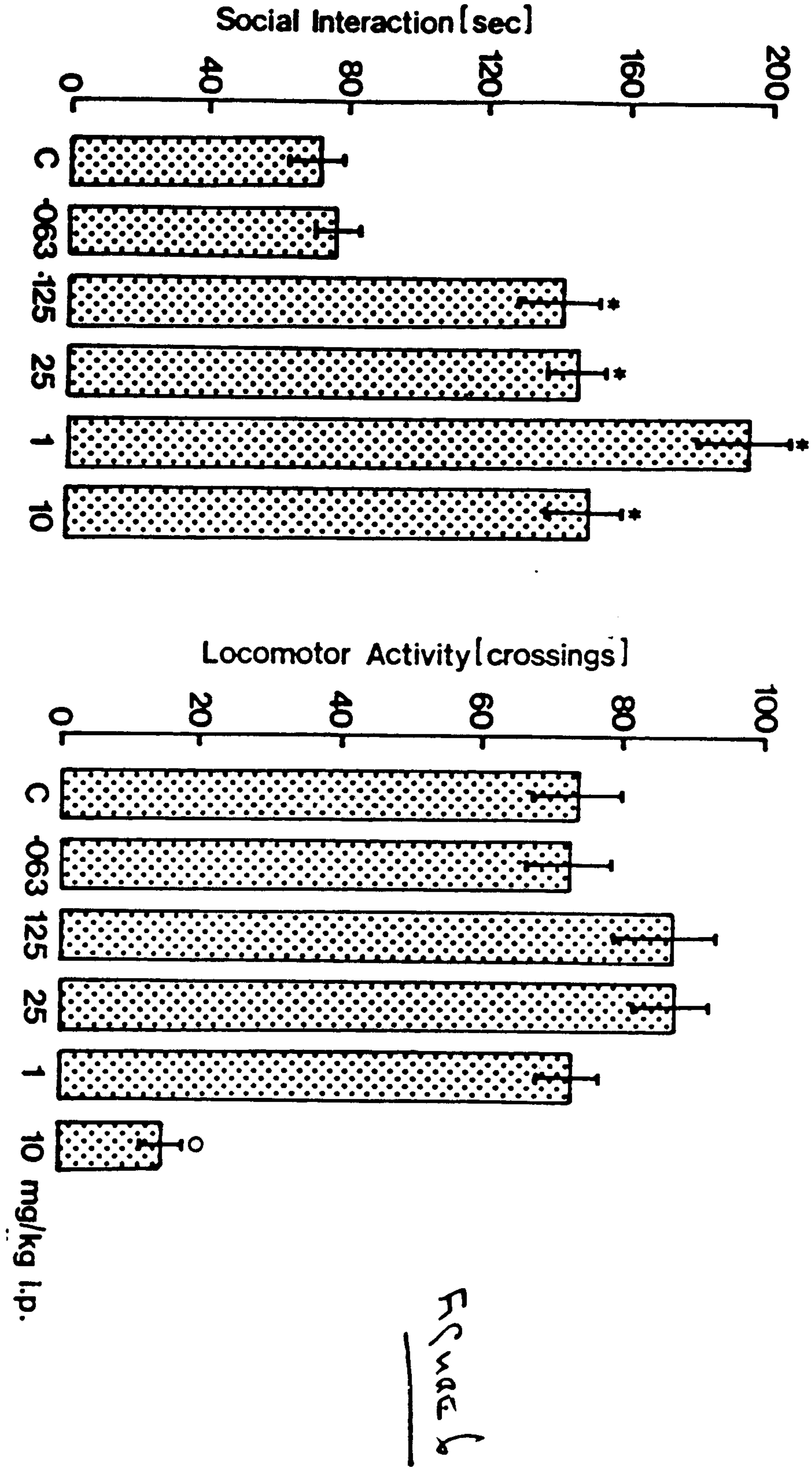
Social Interaction [sec]
0
40
80
120
160
200
C
·063
·125
·25
1
10
Locomotor Activity [crossings]
0
20
40
60
80
100
C
·063
·125
·25
1
10 mg/kg i.p.

Figure 6

FIGURE 7

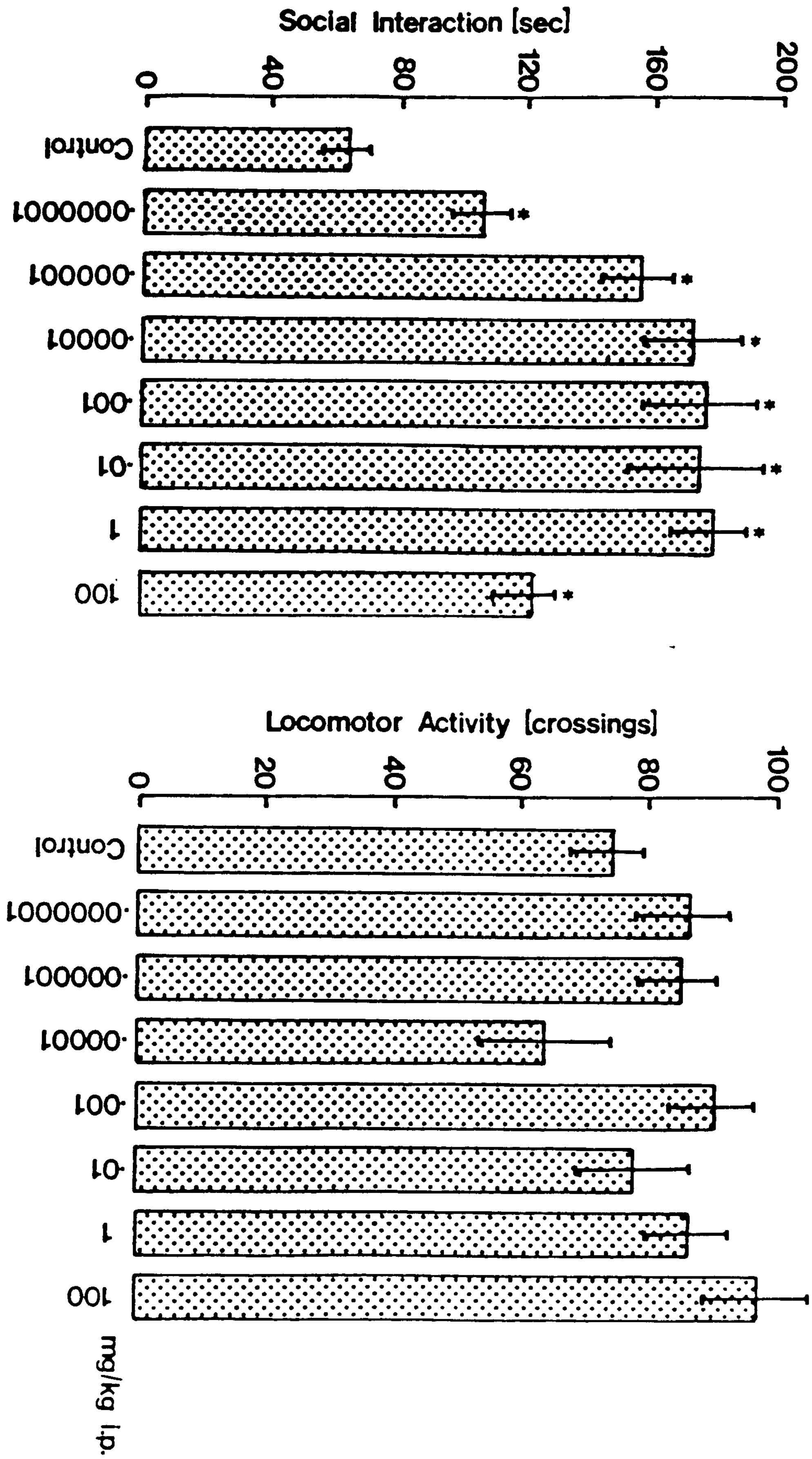

Social Interaction [sec]
0
40
80
120
160
200
Control
.0000001
.000001
.00001
.001
.01
1
100
Locomotor Activity [crossings]
0
20
40
60
80
100
Control
.0000001
.000001
.00001
.001
.01
1
100
mg/kg i.p.

M15ABG

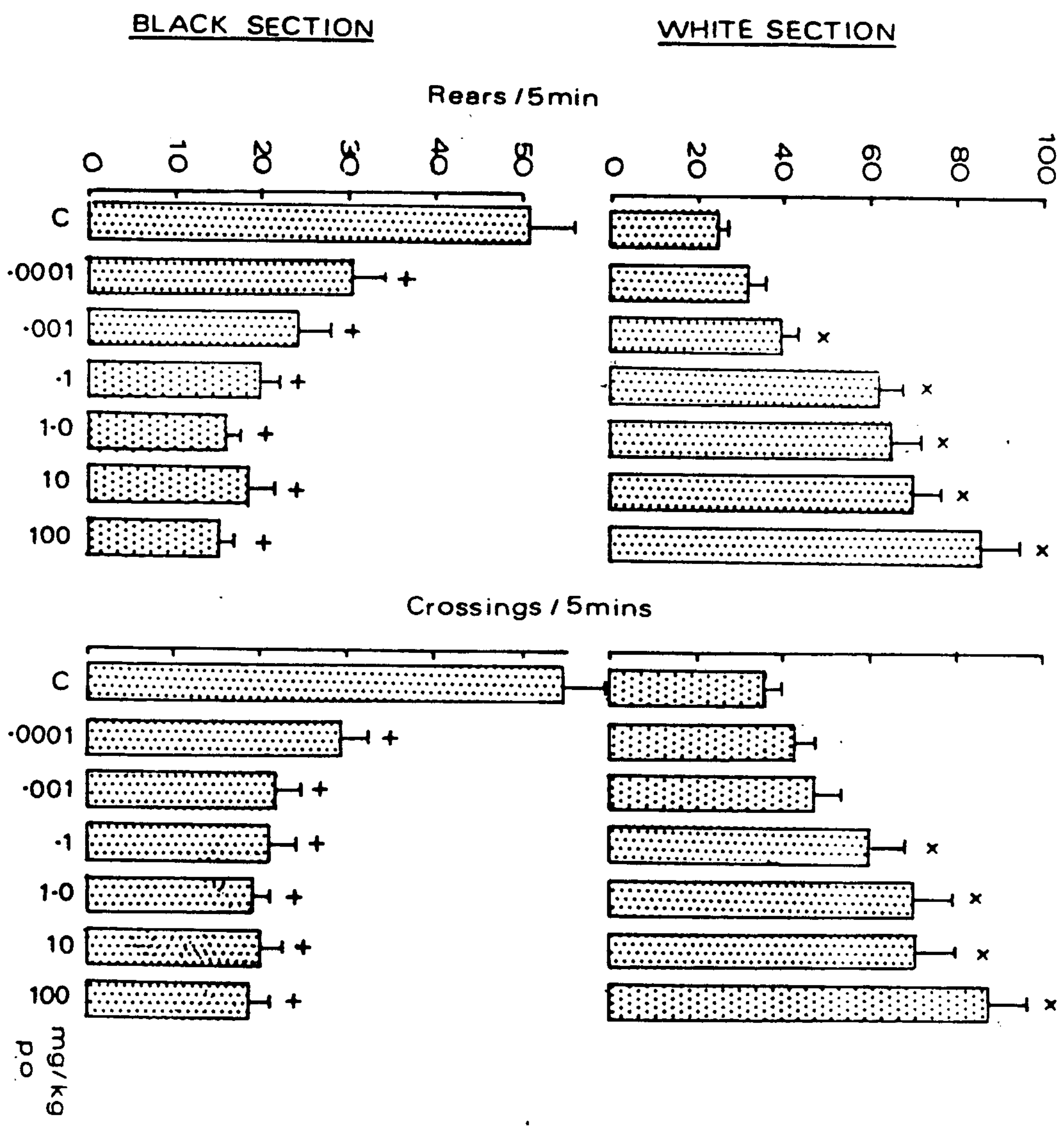


FIGURE 8

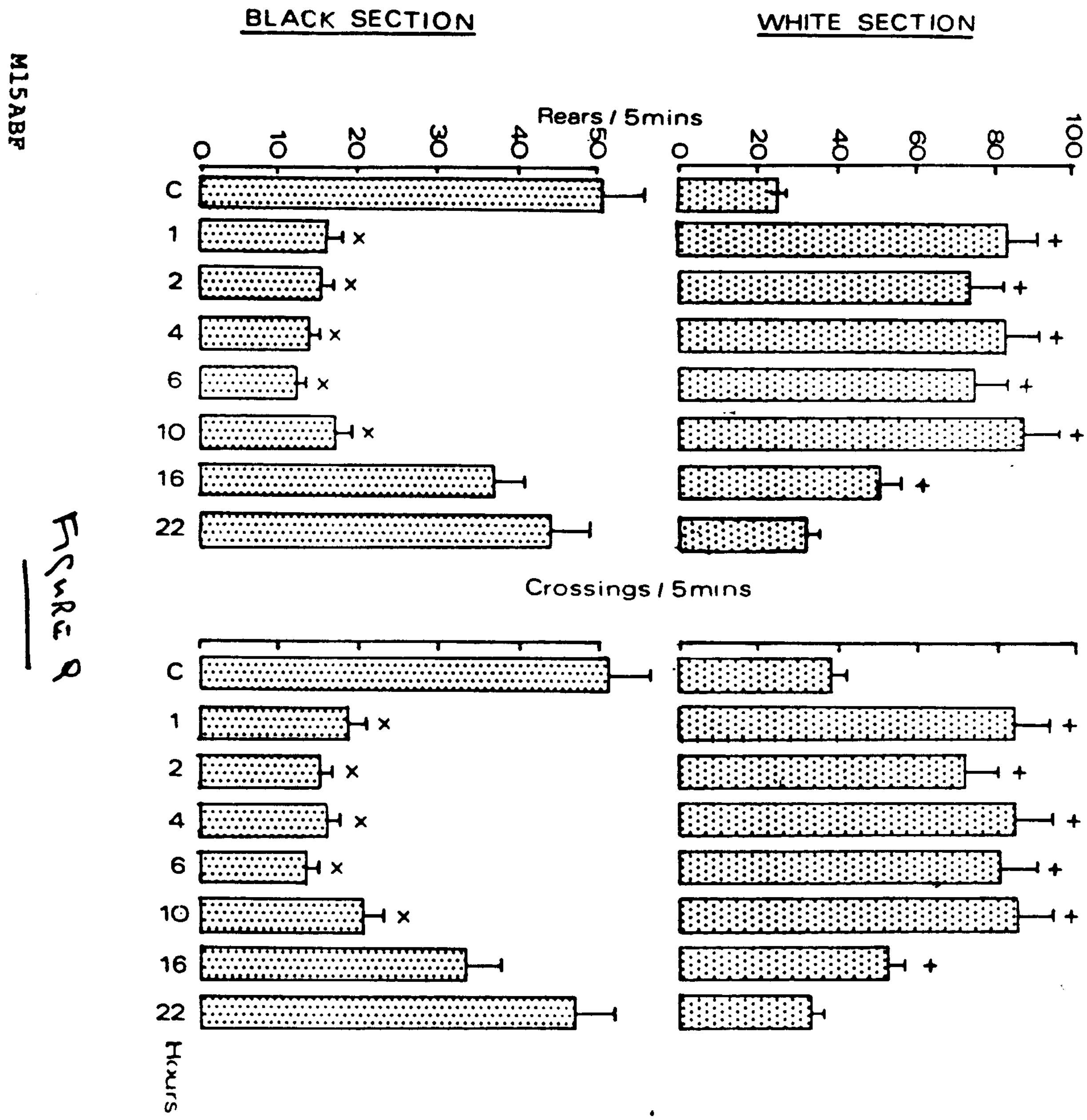
M15ABF
BLACK SECTION
WHITE SECTION
Rears / 5mins
0
10
20
30
40
50
0
20
40
60
80
100
C
1
2
4
6
10
16
22
Crossings / 5mins
Hours
FIGURE 9

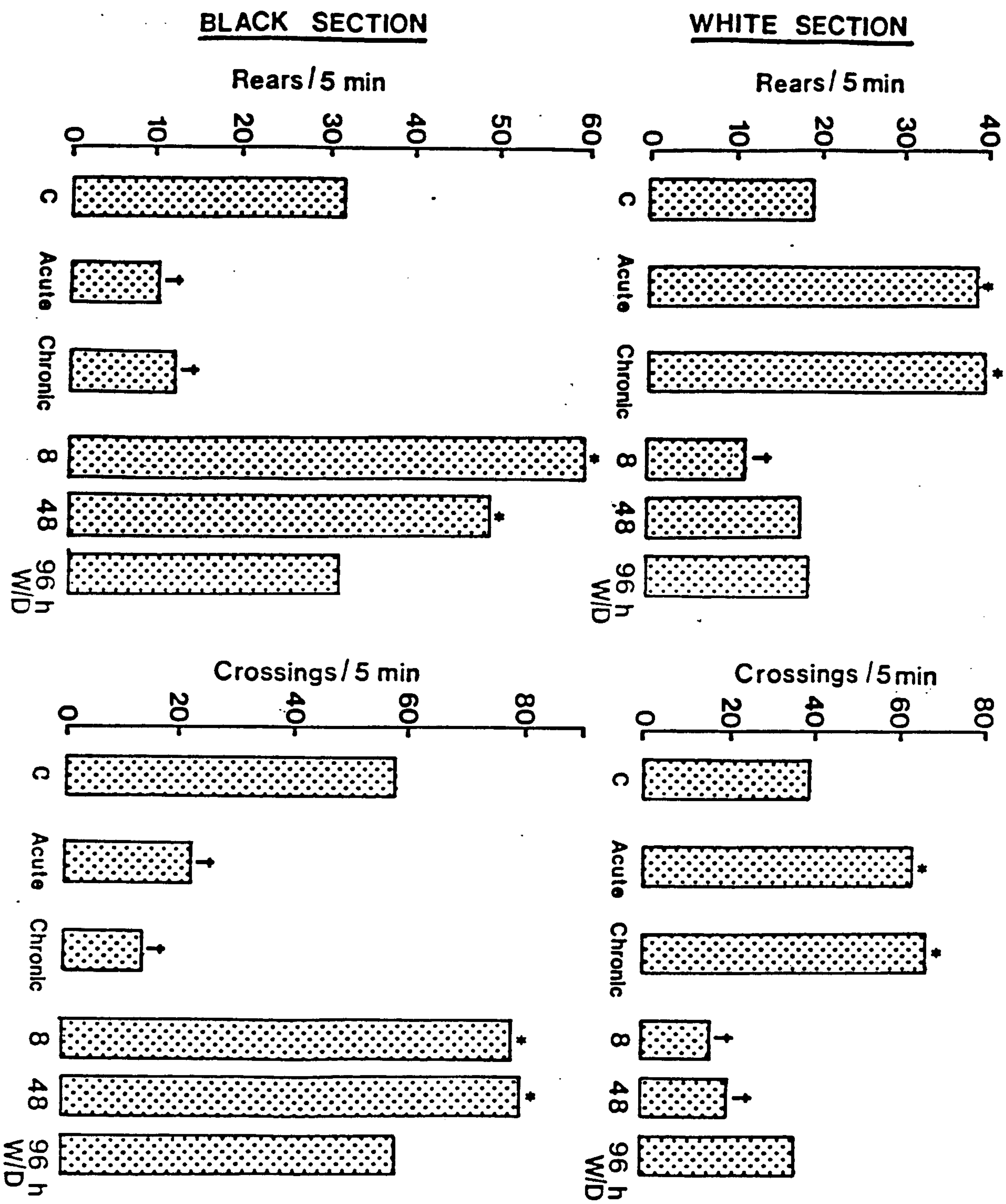
BLACK SECTION
WHITE SECTION
Rears / 5 min
Rears / 5 min
0
10
20
30
40
50
60
0
10
20
30
40
C
Acute
Chronic
8
48
96 h
W/D
Crossings / 5 min
Crossings / 5 min
0
20
40
60
80

FIGURE 10

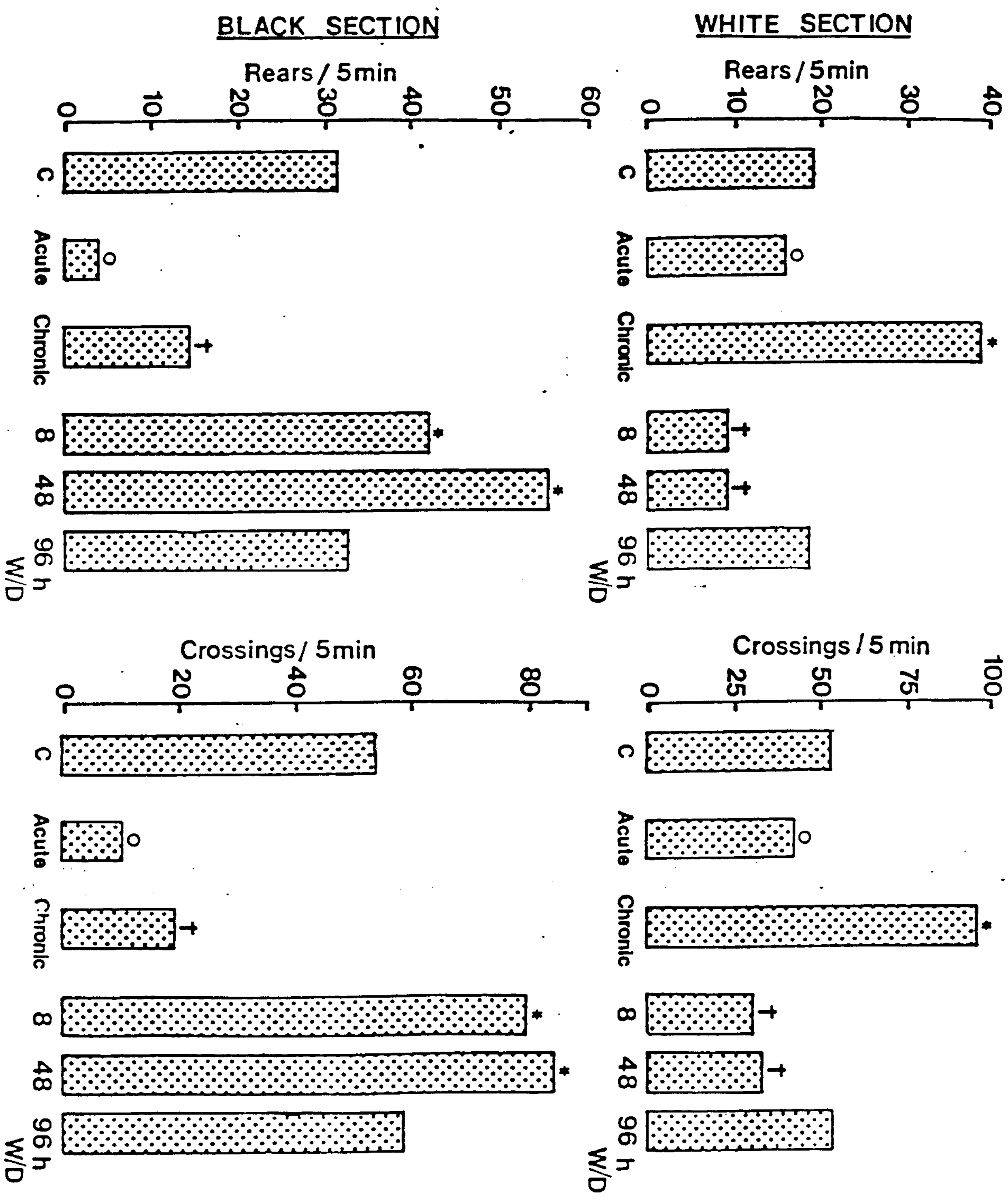
BLACK SECTION
WHITE SECTION
Rears / 5min
0 10 20 30 40 50 60
Rears / 5min
0 10 20 30 40
C
Acute
Chronic
8
48
96 h
W/D
Crossings/ 5min
0 20 40 60 80
Crossings / 5 min
0 25 50 75 100
C
Acute
Chronic
8
48
96 h
W/D

FIGURE 11

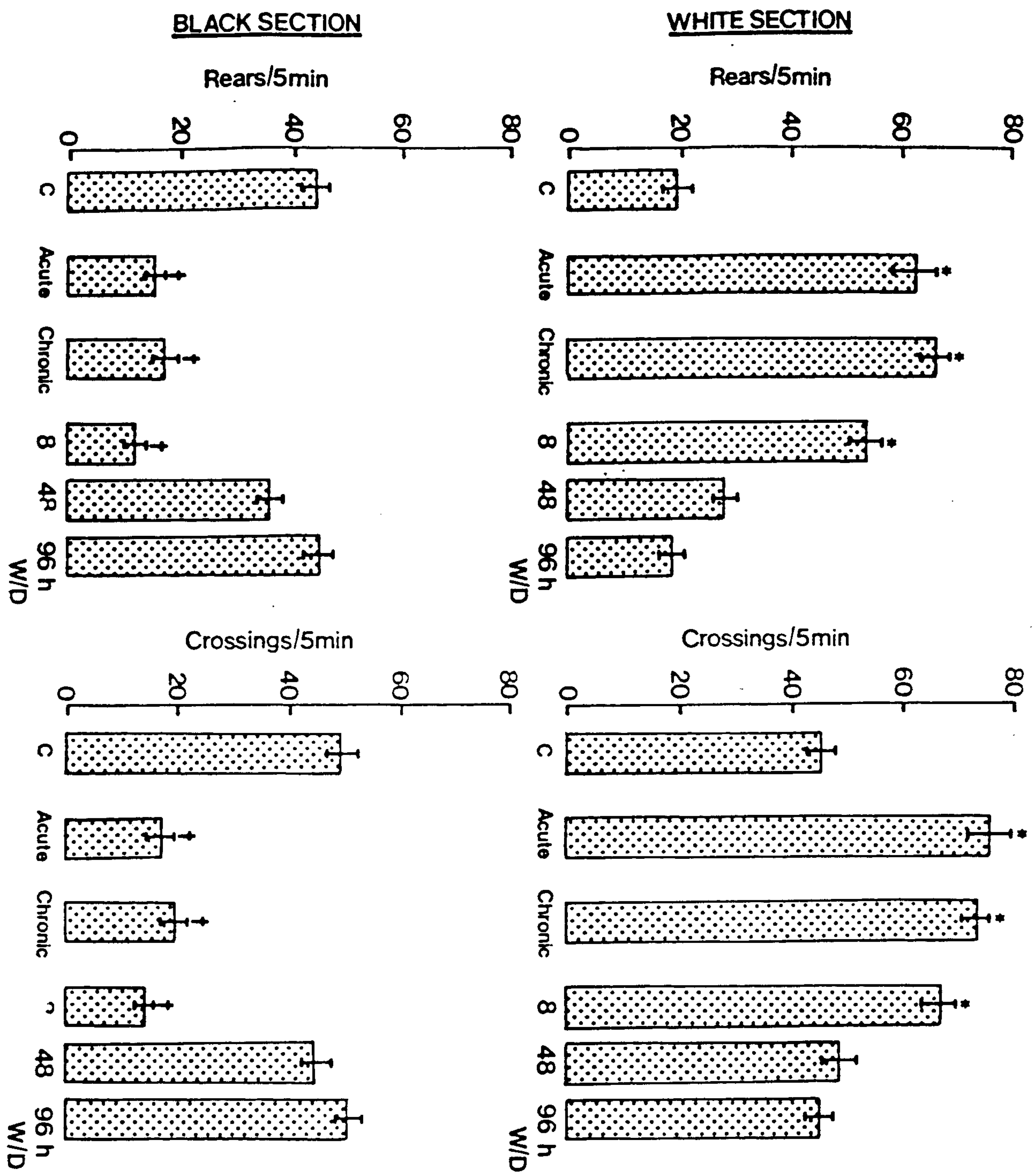
BLACK SECTION
WHITE SECTION
Rears/5min
Crossings/5min
0
20
40
60
80
C
Acute
Chronic
8
48
96 h
W/D

FIGURE 12

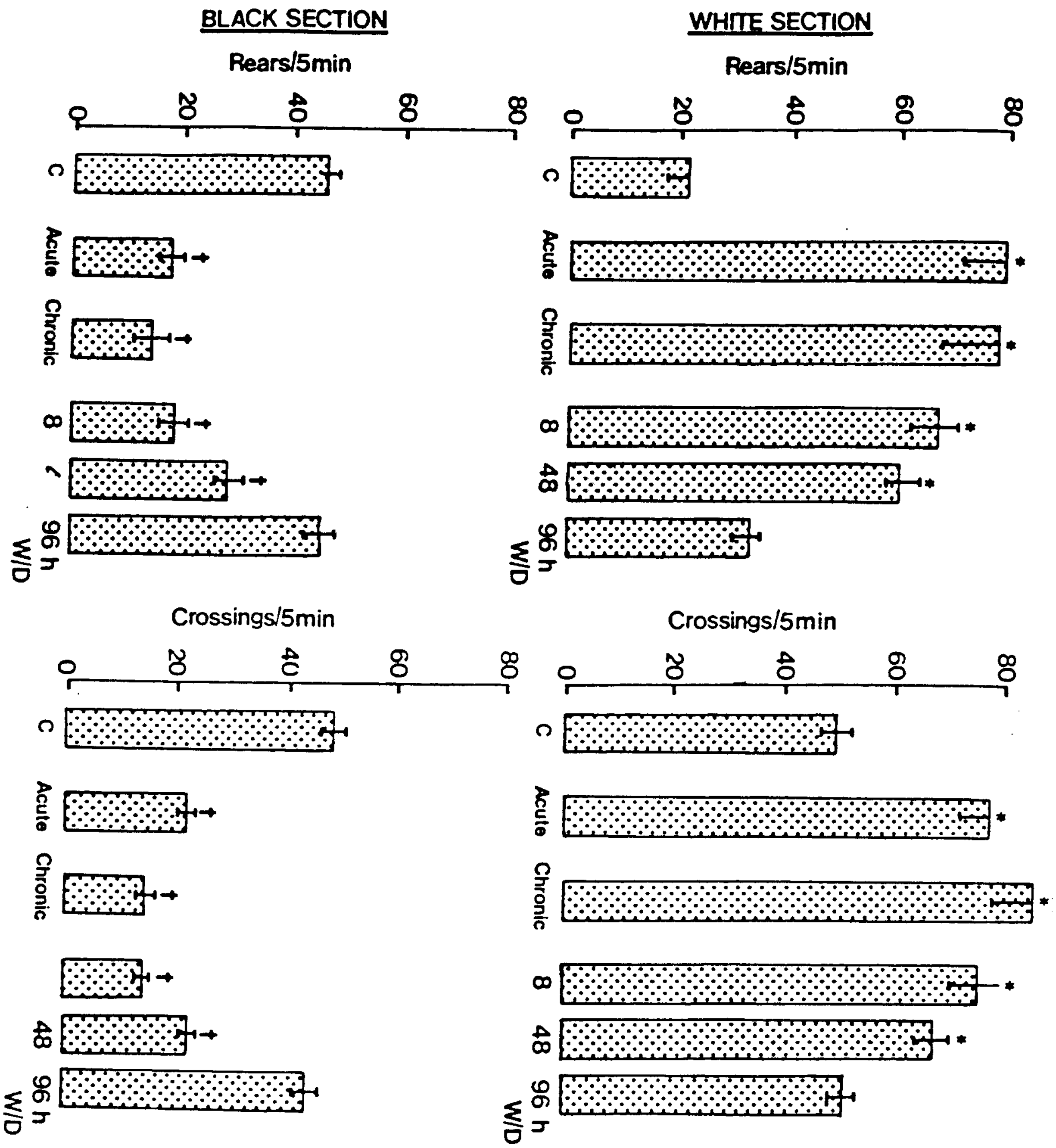
BLACK SECTION
WHITE SECTION
Rears/5min
Rears/5min
Crossings/5min
Crossings/5min
0
20
40
60
80
C
Acute
Chronic
8
48
96 h
W/D

FIGURE 13

FIGURE 14

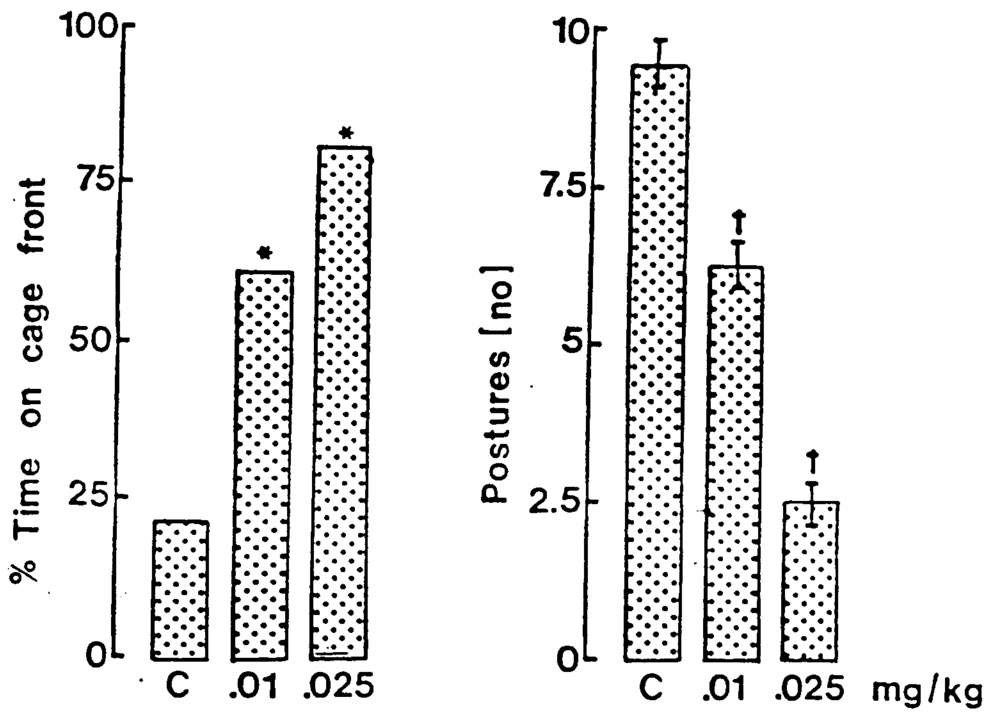
100
75
50
25
0
% Time on cage front
C
.01
.025
10
7.5
5
2.5
0
Postures [no]
C
.01
.025
mg/kg

FIGURE 15

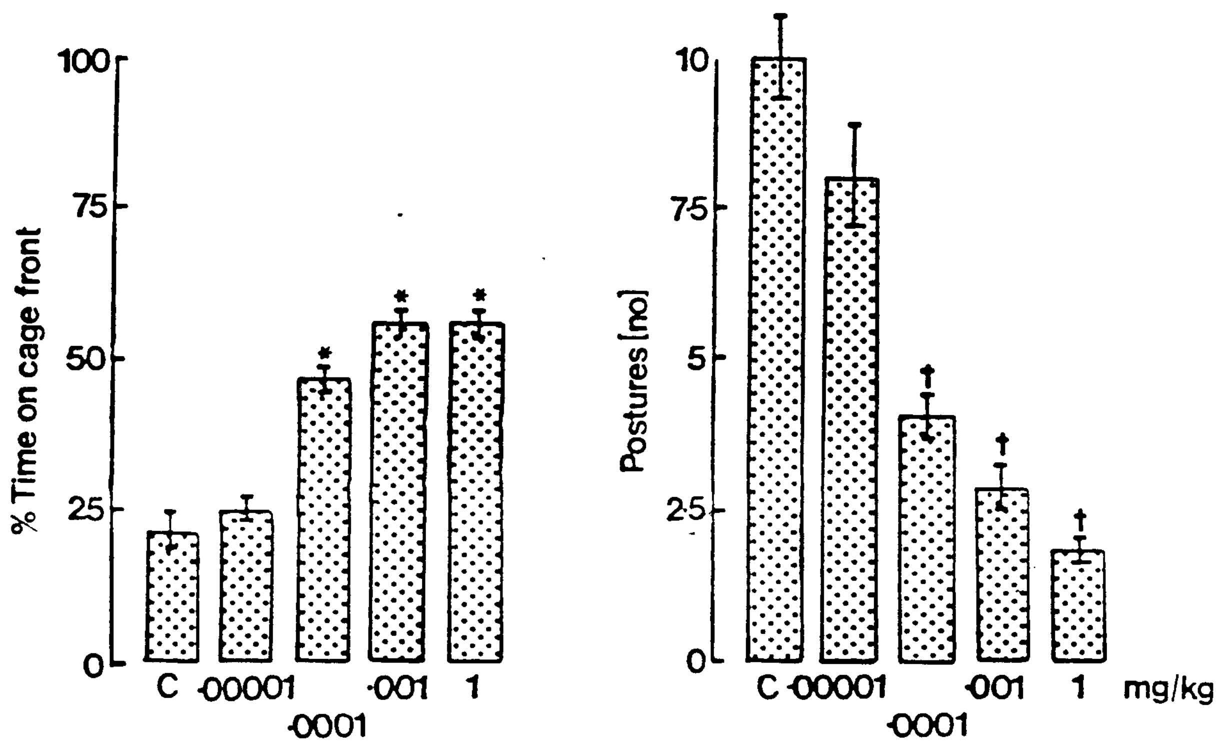
100
75
50
25
0
% Time on cage front
C
·00001
·0001
·001
1
10
7·5
5
2·5
0
Postures [no]
C
·00001
·0001
·001
1
mg/kg

FIGURE 1b

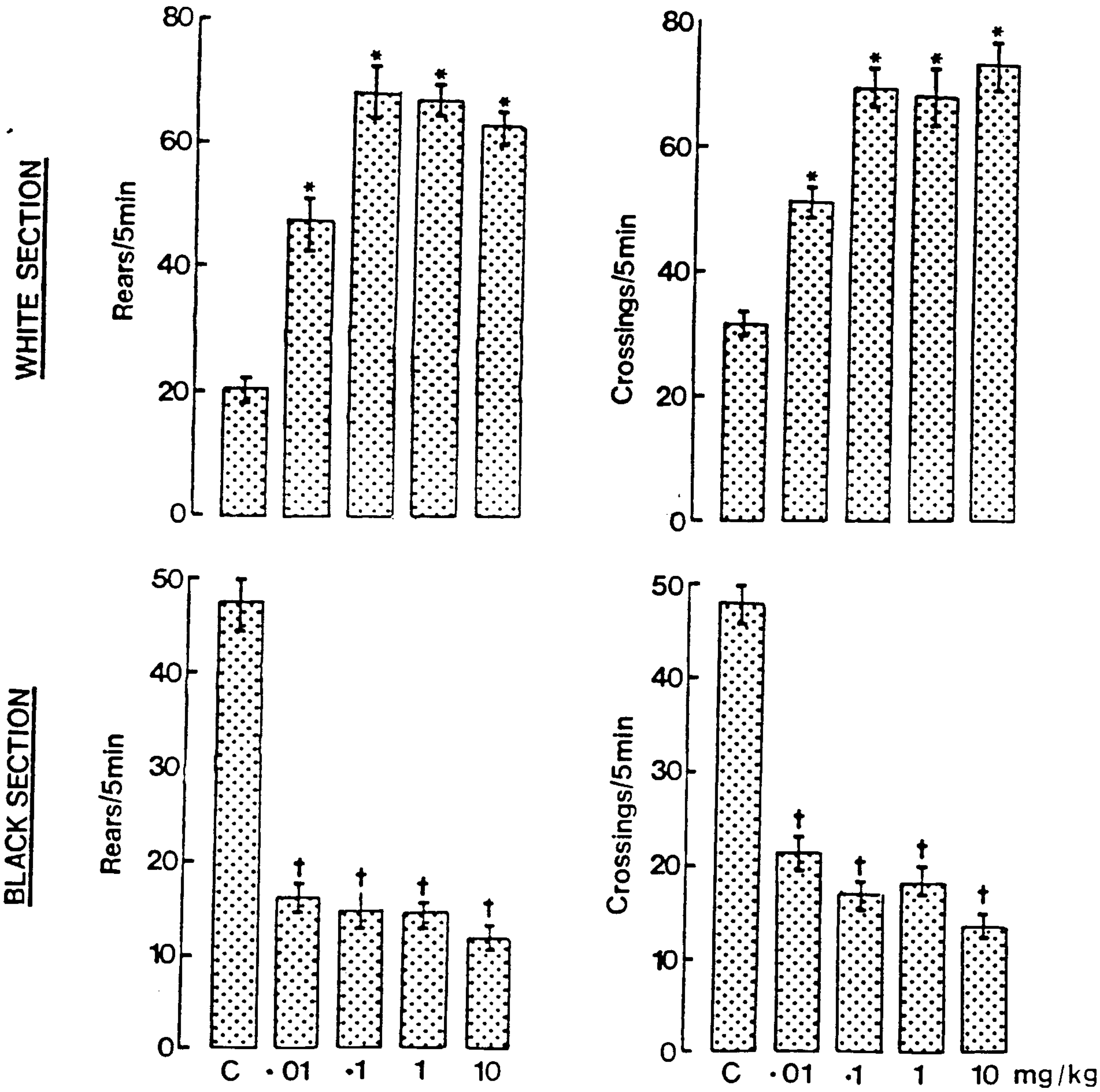
WHITE SECTION
BLACK SECTION
Rears/5min
Crossings/5min
0
20
40
60
80
0
10
20
30
40
50
C
·01
·1
1
10
mg/kg